# EUROPEAN PATENT APPLICATION

(11) **EP 3 650 448 A1**
(43) Date of publication of application: **13.05.2020**
(21) Application number: 18828893.0
(22) Date of filing: 05.07.2018
(51) Int. Cl.: C07D 401/14, C07D 401/06, C07D 401/12, C07D 235/10, C07D 235/12, A61K 31/4184, A61P 37/00, A61P 35/00, A61P 29/00

(54) **INDOLE-FORMAMIDE DERIVATIVE, PREPARATION METHOD THEREFOR AND USE THEREOF IN MEDICINE**

(30) Priority: 06.07.2017 CN 201710546877; 29.08.2017 CN 201710755196; 12.09.2017 CN 201710815286
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: LIU, Dong, Shanghai 200245 (CN); LU, Biao, Shanghai 200245 (CN); QIAN, Wenjian, Shanghai 200245 (CN); DONG, Huaide, Shanghai 200245 (CN); LIU, Suxing, Shanghai 200245 (CN); ZHANG, Rumin, Shanghai 200245 (CN); HE, Feng, Shanghai 200245 (CN); TAO, Weikang, Shanghai 200245 (CN)
(74) Representative: Regimbeau
(86) International application number: PCT/CN2018/094610
(87) International publication number: WO 2019/007382

(57) **Abstract**

Disclosed are an indole-formamide derivative, a preparation method therefor and the use thereof in medicine. In particular, disclosed are an indole-formamide derivative as shown in general formula (I), a preparation method therefor, a pharmaceutical composition containing the derivative, and the use thereof as an ROR agonist and the use thereof for preventing and/or treating a tumour or cancer.

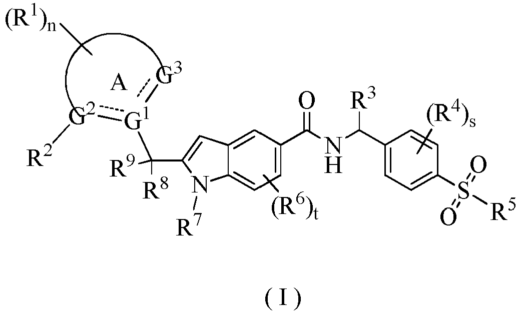

## Description

### FIELD OF THE INVENTION

The present invention belongs to the field of medicine, and relates to an indole-formamide derivative, a method for preparing the same, and a use thereof in medicine. In particular, the present invention relates to an indole-formamide derivative of formula (I), a method for preparing the same, a pharmaceutical composition comprising the same, a use thereof as a ROR agonist, and a use thereof in the preparation of a medicament for preventing and/or treating tumor or cancer.

### BACKGROUND OF THE INVENTION

Retinoid-related orphan receptor (ROR) is a member of the nuclear receptor family, and is also a class of ligand-dependent transcription factors. It can regulate a variety of physiological and biochemical processes, including reproductive development, metabolism, immune system regulation and the like (Mech Dev. 1998 Jan, 70 (1-2: 147-53; EMBO J. 1998 Jul 15, 17(14): 3867-77). The ROR family includes three types: RORα, RORβ and RORγ (Curr Drug Targets Inflamm Allergy. 2004 Dec, 3(4): 395-412), among which, RORγ can be expressed in many tissues, including thymus, liver, kidney, adipose, skeletal muscle and the like (Immunity. 1998 Dec, 9(6):797-806).

RORγ has two subtypes: RORγ1 and RORγ1 (RORγ2), among which, RORγ1 is expressed in many tissues, such as thymus, muscle, kidney and liver, while RORγt is merely expressed in immune cells (Eur J Immunol. 1999 Dec, 29(12):4072-80). It has been reported in the literature that RORyt can regulate the survival of T cells during the differentiation of immune cells, and can activate and promote the differentiation of CD4+ and CD8+ cells into helper T cell 17 (Th17) and cytotoxic T cells (Tc17) (J Immunol. 2014 Mar 15, 192(6):2564-75). TH17 and Tc17 cells are a class of effector cells that promote inflammatory response, enhance acquired immune response and autoimmune response by secreting interleukin-17 (IL-17) and other inflammatory factors such as IL-21. In addition, existing studies have shown that the growth of transplanted tumor can be significantly inhibited by transplanting Th17 cells and Tc17 cells into tumor-bearing mice (J Immunol. 2010 Apr 15, 184(8):4215-27). Th17 can also recruit cytotoxic CD8+ T cells and natural killer cells to enter the tumor microenvironment, thereby killing tumor cells for an anti-tumor purpose (Blood. 2009 Aug 6, 114(6):1141-9; Clin Cancer Res. 2008 Jun 1, 14(11):3254-61). Therefore, activation of RORγt is likely to be a novel anti-tumor therapy.

At present, pharmaceutical companies have developed agonists of RORγt, such as the small molecule drug LYC-55716 developed by Lycera Corp. Pre-clinical studies have shown that its analog LYC-54143 inhibits tumor growth through two distinct pathways, and exhibits a superior anticancer activity. Firstly, LYC-54143 activates RORγt to regulate the differentiation of Th17 and Tc17 cells through traditional pathways, promote the expression of other cytokines such as IL-17, and increase T cell activity. Moreover, activated RORγt can regulate the expression of various genes in the immune system, inhibit the expression of PD-1 in cellular checkpoint receptors, thereby reducing immunosuppression and increasing anticancer activity (Oncoimmunology. 2016 Nov 4, 5(12): e1254854; ACS Chem Biol. 2016 Apr 15, 11(4):1012-8). Although LYC-55716 has currently entered clinical phase II, there are still very few drugs related to this target, and there are no drugs on the market. Disclosed patent applications include, for example, WO2015171558, WO2008152260, WO2007068580, WO2007068579, WO2005056516, WO2005056510, WO2005066116 and WO00228810. There is still a need to continue to develop novel and more efficient RORγt agonists in order to provide the patients with novel and effective anticancer drugs.

The inventors have designed an indole-formamide compound having a structure represented by formula (I) that exhibits a significant effect of agonizing ROR. During the study of ROR agonists, the inventors have also found that in the compound of formula (I) of the present invention, changes of the ortho group of ring A can alter its regulation effect. When the ortho group of ring A is a group having a small steric hindrance (such as H), the compound of formula (I) is an inverse agonist. When the ortho group of ring A is a group having a large steric hindrance, for example haloalkyl (such as trifluoromethyl), alkyl (such as ethyl) and haloalkoxy (such as trifluoromethoxy), the compound of formula (I) is a ROR agonist. The present invention also provides a pharmacodynamic test, in which the compound of the present invention exhibits a good antitumor activity when being administrated alone. In addition, the compound of the present invention exhibits a synergistic effect when being administrated in combination with a PD-1 antibody, leading to a novel way of improving the efficacy of immunotherapy.

### SUMMARY OF THE INVENTION

The object of the present invention is to provide a compound of formula (I): 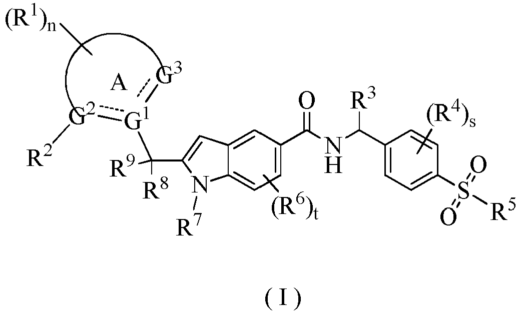 or a tautomer, mesomer, racemate, enantiomer, diastereomer thereof, or mixture thereof, or a pharmaceutically acceptable salt thereof,
wherein:
----- is a double bond or single bond;
G¹, G² and G³ are identical or different and are each independently selected from the group consisting of C, CH, CH₂ and N;
ring A is selected from the group consisting of aryl, heteroaryl, cycloalkyl and heterocyclyl;
each R¹ is identical or different and each is independently selected from the group consisting of hydrogen, halogen, alkyl, haloalkyl, alkoxy, haloalkoxy, cyano, amino, nitro, hydroxy and hydroxyalkyl;
R² is a haloalkyl;
R³ is selected from the group consisting of alkyl, haloalkyl, alkoxy, haloalkoxy, hydroxyalkyl, halogen, cyano, amino, nitro, hydroxy, cycloalkyl, heterocyclyl, aryl and heteroaryl, wherein the alkyl, haloalkyl, cycloalkyl, heterocyclyl, aryl and heteroaryl are each independently optionally substituted by one or more substituents selected from the group consisting of hydroxy, halogen, alkyl, alkoxy and amino;
each R⁴ is identical or different and each is independently selected from the group consisting of hydrogen, halogen, alkyl, haloalkyl, alkoxy, haloalkoxy, cyano, amino, nitro, hydroxy, hydroxyalkyl, cycloalkyl, heterocyclyl, aryl and heteroaryl;
R⁵ is selected from the group consisting of hydrogen, alkyl, haloalkyl, amino, hydroxy, hydroxyalkyl, cycloalkyl, heterocyclyl, NR¹⁰R¹¹, aryl and heteroaryl, wherein the alkyl, cycloalkyl, heterocyclyl, aryl and heteroaryl are each independently optionally substituted by one or more substituents selected from the group consisting of hydroxy, halogen, alkyl, amino, cycloalkyl and heterocyclyl;
each R⁶ is identical or different and each is independently selected from the group consisting of hydrogen, halogen, alkyl, haloalkyl, alkoxy, haloalkoxy, cyano, amino, nitro, hydroxy, hydroxyalkyl, cycloalkyl, heterocyclyl, aryl and heteroaryl;
R⁷ is selected from the group consisting of hydrogen, alkyl, haloalkyl, cycloalkyl and heterocyclyl, wherein the alkyl is optionally substituted by one or more substituents selected from the group consisting of halogen, nitro, cycloalkyl and heterocyclyl;
R⁸ and R⁹ are identical or different and are each independently selected from the group consisting of hydrogen, halogen, alkyl, haloalkyl, alkoxy, cyano, amino, nitro, hydroxy and hydroxyalkyl;
R¹⁰ and R¹¹ are identical or different and are each independently selected from the group consisting of hydrogen, alkyl, haloalkyl, amino, hydroxy, hydroxyalkyl, cycloalkyl, heterocyclyl, aryl and heteroaryl;
n is 0, 1, 2, 3 or 4;
s is 0, 1, 2 or 3; and
t is 0, 1, 2 or 3.

In a preferred embodiment of the present invention, the compound of formula (I) is a compound of formula (II): 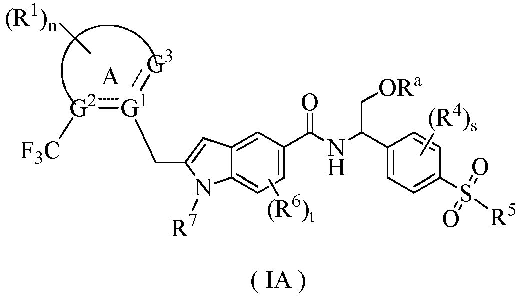 wherein:
R^{a} is hydrogen or alkyl;
-----, ring A, G¹∼G³, R¹, R⁴∼R⁷, n, s and t are as defined in formula (I).

In a preferred embodiment of the present invention, the compound of formula (I) is a compound of formula (II): 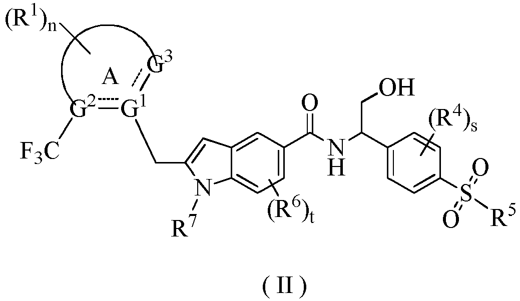 wherein:
-----, ring A, G¹∼G³, R¹, R⁴∼R⁷, n, s and t are as defined in formula (I).

In a preferred embodiment of the present invention, the compound of formula (I) is a compound of formula (I') below: 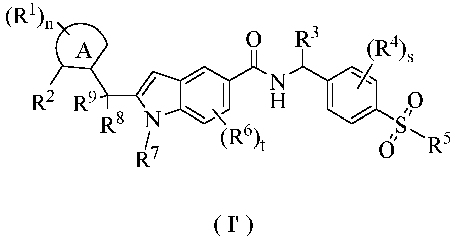 ring A, R¹∼R⁹, n, s and t are as defined in formula (I).

In another preferred embodiment of the present invention, the compound of formula (II) is a compound of formula (II') below: 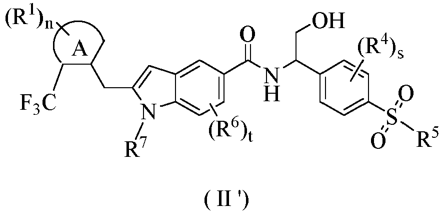 ring A, R¹, R⁴∼R⁷, n, s and t are as defined in formula (II).

In a preferred embodiment of the present invention, in the compound of formula (I), ring A is selected from the group consisting of phenyl, pyridyl, imidazolyl, pyrazolyl and morpholinyl.

In a preferred embodiment of the present invention, in the compound of formula (I), 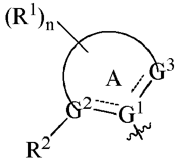 is selected from the group consisting of 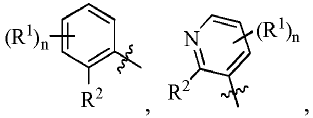 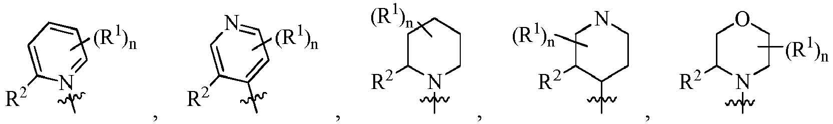 and

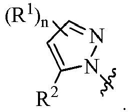

In a preferred embodiment of the present invention, the compound of formula (I) is a compound of formula (III): 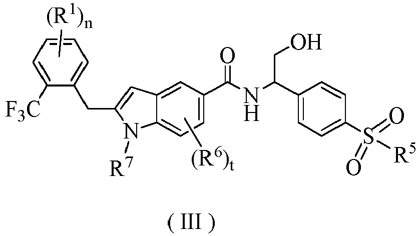 wherein:
R¹, R⁵∼R⁷, n and t are as defined in formula (I).

In a preferred embodiment of the present invention, the compound of formula (I) is a compound of formula (IV): 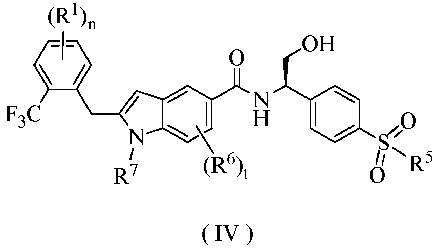 wherein:
R¹, R⁵∼R⁷, n and t are as defined in formula (I).

In a preferred embodiment of the present invention, in the compound of formula (I), R¹ is selected from the group consisting of hydrogen, halogen and alkyl.

In a preferred embodiment of the present invention, in the compound of formula (I), R⁵ is selected from the group consisting of alkyl, NR¹⁰R¹¹ and cycloalkyl, wherein the alkyl and cycloalkyl are each independently optionally substituted by one or more substituents selected from the group consisting of hydroxy, halogen, alkyl, amino, cycloalkyl and heterocyclyl; R¹⁰ and R¹¹ are as defined in formula (I).

In a preferred embodiment of the present invention, in the compound of formula (I), R⁵ is selected from the group consisting of ethyl, cyclopropyl, cyclopropylmethyl and -NH-cyclopropyl.

In a preferred embodiment of the present invention, in the compound of formula (I), R⁶ is hydrogen or halogen.

In a preferred embodiment of the present invention, in the compound of formula (I), R⁷ is selected from the group consisting of alkyl, cycloalkyl and haloalkyl.

Typical compounds of formula (I) include, but are not limited to:

| Example No. | Structure and name of the compound |
|---|---|
| 1 | 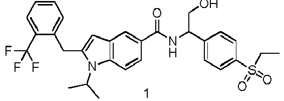 |
| | *N-*(1-(4-(Ethylsulfonyl)phenyl)-2-hydroxyethyl)-1-isopropyl-2-(2-(trifluo romethyl)benzyl)-1*H*-indole-5-carboxamide **1** |
| 2 | 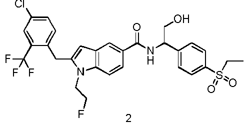 |
| | 2-(4-Chloro-2-(trifluoromethyl)benzyl)-*N*-(1-(4-(ethylsulfonyl)phenyl)-2 -hydroxyethyl)-1-(2-fluoroethyl)-1*H*-indole-5-carboxamide **2** |
| 3 | 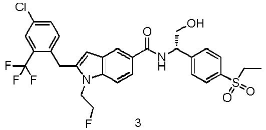 |
| | (*S*)-2-(4-Chloro-2-(trifluoromethyl)benzyl)-*N*-(1-(4-(ethylsulfonyl)pheny 1)-2-hydroxyethyl)-1-(2-fluoroethyl)-1*H*-indole-5-carboxamide **3** |
| 4 | 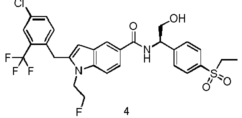 |
| | (*R*)-2-(4-Chloro-2-(trifluoromethyl)benzyl)-*N*-(1-(4-(ethylsulfonyl)pheny l)-2-hydroxyethyl)-1-(2-fluoroethyl)-1*H*-indole-5-carboxamide **4** |
| 5 | 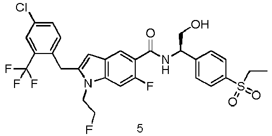 |
| | (*R*)-2-(4-Chloro-2-(trifluoromethyl)benzyl)-*N*-(1-(4-(ethylsulfonyl)pheny l)-2-hydroxyethyl)-6-fluoro-1-(2-fluoroethyl)-1*H*-indole-5-carboxamide **5** |
| 6 | 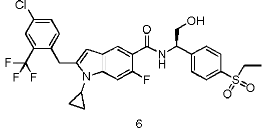 |
| | (*R*)-2-(4-Chloro-2-(trifluoromethyl)benzyl)-1-cyclopropyl-*N*-(1-(4-(ethyl sulfonyl)phenyl)-2-hydroxyethyl)-6-fluoro-1*H*-indole-5-carboxamide **6** |
| 7 | 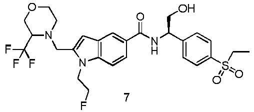 |
| | *N*-((*R*)-1-(4-(Ethylsulfonyl)phenyl)-2-hydroxyethyl)-1-(2-fluoroethyl)-2-((3-(trifluoromethyl)morpholino)methyl)-1*H*-indole-5-carboxamide **7** |
| 8 | 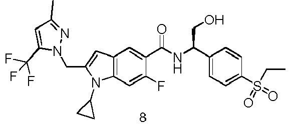 |
| | (*R*)-1-Cyclopropyl-*N*-(1-(4-(ethylsulfonyl)phenyl)-2-hydroxyethyl)-6-flu oro-2-((3-methyl-5-(trifluoromethyl)-1*H*-pyrazol-1-yl)methyl)-1*H*-indole -5-carboxamide **8** |
| 9 | 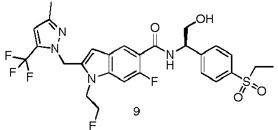 |
| | (*R*)-*N*-(1-(4-(Ethylsulfonyl)phenyl)-2-hydroxyethyl)-6-fluoro-1-(2-fluoro ethyl)-2-((3-methyl-5-(trifluoromethyl)-1*H-*pyrazol-1-yl)methyl)-1*H-*ind ole-5-carboxamide **9** |
| 10 | 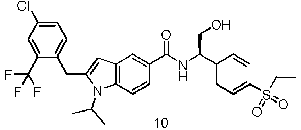 |
| | (*R*)-2-(4-Chloro-2-(trifluoromethyl)benzyl)-*N*-(1-(4-(ethylsulfonyl)pheny l)-2-hydroxyethyl)-1-isopropyl-1*H*-indole-5-carboxamide **10** |
| 11 | 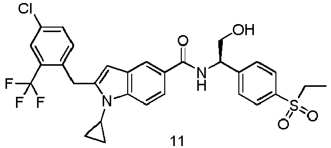 |
| | (*R*)-2-(4-Chloro-2-(trifluoromethyl)benzyl)-1-cyclopropyl-*N*-(1-(4-(ethyl sulfonyl)phenyl)-2-hydroxyethyl)-1*H*-indole-5-carboxamide **11** |
| 12 | 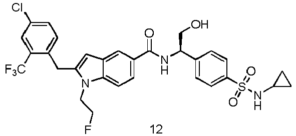 |
| | (*R*)-2-(4-Chloro-2-(trifluoromethyl)benzyl)-*N*-(1-(4-(*N*-cyclopropylsulfa moyl)phenyl)-2-hydroxyethyl)-1-(2-fluoroethyl)-1*H*-indole-5-carboxami de **12** |
| 13 | 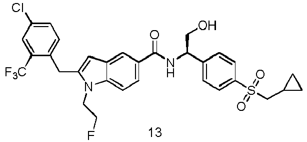 |
| | (*R*)-2-(4-Chloro-2-(trifluoromethyl)benzyl)-*N*-(1-(4-((cyclopropylmethyl )sulfonyl)phenyl)-2-hydroxyethyl)-1-(2-fluoroethyl)-1*H*-indole-5-carbox amide **13** |
| 14 | 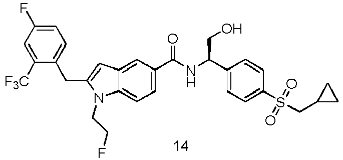 |
| | (*R*)-*N*-(1-(4-((Cyclopropylmethyl)sulfonyl)phenyl)-2-hydroxyethyl)-2-(4 -fluoro-2-(trifluoromethyl)benzyl)-1-(2-fluoroethyl)-1*H*-indole-5-carbox amide **14** |
| 15 | 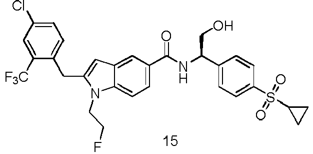 |
| | (*R*)-2-(4-Chloro-2-(trifluoromethyl)benzyl)-*N*-(1-(4-(cyclopropylsulfonyl )phenyl)-2-hydroxyethyl)-1-(2-fluoroethyl)-1*H*-indole-5-carboxamide **15** |
| 16 | 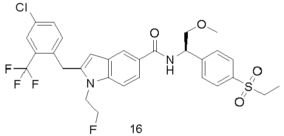 |
| | (*R*)-2-(4-Chloro-2-(trifluoromethyl)benzyl)-*N*-(1-(4-(ethylsulfonyl)pheny l)-2-methoxyethyl)-1-(2-fluoroethyl)-1*H*-indole-5-carboxamide **16** |

or a tautomer, mesomer, racemate, enantiomer, diastereomer thereof, or mixture thereof, or a pharmaceutically acceptable salt thereof.

In another aspect, the present invention relates to a pharmaceutical composition comprising a therapeutically effective amount of the compound of formula (I), or a tautomer, mesomer, racemate, enantiomer, diastereomer thereof, or mixture thereof, or a pharmaceutically acceptable salt thereof, and one or more pharmaceutically acceptable carriers, diluents or excipients. The present invention also relates to a method for preparing the pharmaceutical composition, comprising a step of mixing the compound of formula (I), or a tautomer, mesomer, racemate, enantiomer, diastereomer thereof, or mixture thereof, or a pharmaceutically acceptable salt thereof with the pharmaceutically acceptable carrier(s), diluent(s) or excipient(s). In an embodiment of the present invention, the pharmaceutical composition further comprises an anti-PD-1 antibody, preferably an anti-mouse PD-1 antibody.

The present invention further relates to a use of the compound of formula (I), or a tautomer, mesomer, racemate, enantiomer, diastereomer thereof, or mixture thereof, or a pharmaceutically acceptable salt thereof, or the pharmaceutical composition comprising the same in the preparation of a ROR agonist.

The present invention further relates to a use of the compound of formula (I), or a tautomer, mesomer, racemate, enantiomer, diastereomer thereof, or mixture thereof, or a pharmaceutically acceptable salt thereof, or the pharmaceutical composition comprising the same as a ROR agonist in the preparation of a medicament for preventing and/or treating tumor or cancer.

The present invention further relates to a use of the compound of formula (I), or a tautomer, mesomer, racemate, enantiomer, diastereomer thereof, or mixture thereof, or a pharmaceutically acceptable salt thereof (as a ROR agonist), or the pharmaceutical composition comprising the same, in combination with an anti-PD-1 antibody in the preparation of a medicament for preventing and/or treating tumor or cancer.

The present invention further relates to the compound of formula (I), or a tautomer, mesomer, racemate, enantiomer, diastereomer thereof, or mixture thereof, or a pharmaceutically acceptable salt thereof, or the pharmaceutical composition comprising the same, for use as a medicament.

The present invention also relates to the compound of formula (I), or a tautomer, mesomer, racemate, enantiomer, diastereomer thereof, or mixture thereof, or a pharmaceutically acceptable salt thereof, or the pharmaceutical composition comprising the same, for use as a ROR agonist.

The present invention also relates to the compound of formula (I), or a tautomer, mesomer, racemate, enantiomer, diastereomer thereof, or mixture thereof, or a pharmaceutically acceptable salt thereof, or the pharmaceutical composition comprising the same, for use as a ROR agonist in preventing and/or treating tumor or cancer.

The present invention also relates to the combination of the compound of formula (I), or a tautomer, mesomer, racemate, enantiomer, diastereomer thereof, or mixture thereof, or a pharmaceutically acceptable salt thereof, or the pharmaceutical composition comprising the same and an anti-PD-1 antibody, for use in preventing and/or treating tumor or cancer.

The present invention also relates to a method for preventing and/or treating tumor or cancer, comprising a step of administrating to a patient in need thereof a therapeutically effective dose of the compound of formula (I), or a tautomer, mesomer, racemate, enantiomer, diastereomer thereof, or mixture thereof, or a pharmaceutically acceptable salt thereof, or the pharmaceutical composition comprising the same as a ROR agonist.

The present invention also relates to a method for preventing and/or treating tumor or cancer, comprising a step of administrating to a patient in need thereof a therapeutically effective dose of the compound of formula (I), or a tautomer, mesomer, racemate, enantiomer, diastereomer thereof, or mixture thereof, or a pharmaceutically acceptable salt thereof, or the pharmaceutical composition comprising the same and an anti-PD-1 antibody.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such a composition can contain one or more ingredients selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the manufacture of tablets. These excipients can be inert excipients, granulating agents, disintegrating agents, binders and lubricants. The tablet can be uncoated or coated by means of a known technique to mask drug taste or delay the disintegration and absorption of the active ingredient in the gastrointestinal tract, thereby providing sustained release over a long period of time.

An oral formulation can also be provided as soft gelatin capsules in which the active ingredient is mixed with an inert solid diluent, or the active ingredient is mixed with a water-soluble carrier or an oil medium.

An aqueous suspension contains the active ingredient in admixture with excipients suitable for the manufacture of an aqueous suspension. Such excipients are suspending agents, dispersants or wetting agents. The aqueous suspension can also contain one or more preservatives, one or more colorants, one or more flavoring agents, and one or more sweeteners.

An oil suspension can be formulated by suspending the active ingredient in a vegetable oil or mineral oil. The oil suspension can contain a thickener. The aforementioned sweeteners and flavoring agents can be added to provide a palatable formulation. These compositions can be preserved by adding an antioxidant.

The pharmaceutical composition of the present invention can also be in the form of an oil-in-water emulsion. The oil phase can be a vegetable oil, or a mineral oil, or a mixture thereof. Suitable emulsifying agents can be naturally occurring phospholipids. The emulsion can also contain a sweetening agent, flavoring agent, preservative and antioxidant. Such a formulation can also contain a demulcent, preservative, colorant and antioxidant.

The pharmaceutical composition of the present invention can be in the form of a sterile injectable aqueous solution. Acceptable vehicles or solvents that can be used are water, Ringer's solution or isotonic sodium chloride solution. The sterile injectable formulation can be a sterile injectable oil-in-water micro-emulsion in which the active ingredient is dissolved in the oil phase. The injectable solution or micro-emulsion can be introduced into a patient's bloodstream by local bolus injection. Alternatively, the solution and micro-emulsion are preferably administrated in a manner that maintains a constant circulating concentration of the compound of the present invention. In order to maintain this constant concentration, a continuous intravenous delivery device can be used. An example of such a device is Deltec CADD-PLUS. TM. 5400 intravenous injection pump.

The pharmaceutical composition of the present invention can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent. Moreover, sterile fixed oils can easily be used as a solvent or suspending medium. For this purpose, any blended fixed oil can be used. In addition, fatty acids can also be used to prepare injections.

The compound of the present invention can be administrated in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug.

It is well known to those skilled in the art that the dosage of a drug depends on a variety of factors including but not limited to, the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of formula (I) or the type of pharmaceutically acceptable salt thereof can be verified by traditional therapeutic regimens.

### DEFINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, *n*-propyl, isopropyl, *n*-butyl, isobutyl, *tert*-butyl, *sec*-butyl, *n-*pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1 -ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "alkoxy" refers to an -O-(alkyl) or an -O-(unsubstituted cycloalkyl) group, wherein the alkyl and cycloalkyl are as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "cycloalkyl" refers to a saturated or partially unsaturated monocyclic or polycyclic hydrocarbon substituent group having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably 6 to 14 membered spiro cycloalkyl, and more preferably 7 to 10 membered spiro cycloalkyl. According to the number of the spiro atoms shared between the rings, the spiro cycloalkyl can be divided into mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

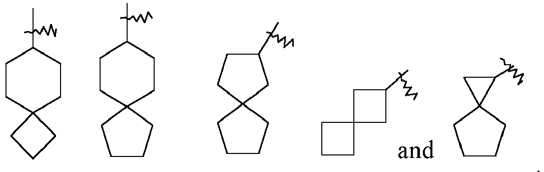

The term "fused cycloalkyl" refers to a 5 to 20 membered all-carbon polycyclic group, wherein each ring in the system shares an adjacent pair of carbon atoms with another ring, wherein one or more rings can contain one or more double bonds, but none of the rings has a completely conjugated π-electron system. The fused cycloalkyl is preferably 6 to 14 membered fused cycloalkyl, and more preferably 7 to 10 membered fused cycloalkyl. According to the number of membered rings, the fused cycloalkyl can be divided into bicyclic, tricyclic, tetracyclic or polycyclic fused cycloalkyl, and the fused cycloalkyl is preferably bicyclic or tricyclic fused cycloalkyl, and more preferably 5-membered/5-membered, or 5-membered/6-membered bicyclic fused cycloalkyl. Non-limiting examples of fused cycloalkyl include:

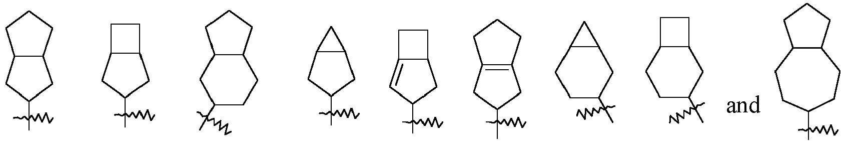

The term "bridged cycloalkyl" refers to a 5 to 20 membered all-carbon polycyclic group, wherein every two rings in the system share two disconnected carbon atoms, wherein the rings can have one or more double bonds, but none of the rings has a completely conjugated π-electron system. The bridged cycloalkyl is preferably 6 to 14 membered bridged cycloalkyl, and more preferably 7 to 10 membered bridged cycloalkyl. According to the number of membered rings, the bridged cycloalkyl can be divided into bicyclic, tricyclic, tetracyclic or polycyclic bridged cycloalkyl, and the bridged cycloalkyl is preferably bicyclic, tricyclic or tetracyclic bridged cycloalkyl, and more preferably bicyclic or tricyclic bridged cycloalkyl. Non-limiting examples of bridged cycloalkyl include:

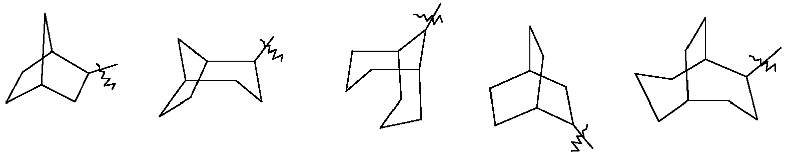

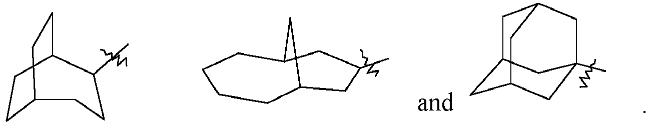

The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "heterocyclyl" refers to a 3 to 20 membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 8 ring atoms wherein 1 to 3 atoms are heteroatoms; and most preferably 3 to 6 ring atoms wherein 1 to 2 atoms are heteroatoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably piperidinyl, piperazinyl or morpholinyl. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O and S(O)ₘ (wherein m is an integer of 0 to 2), with the remaining ring atoms being carbon atoms, where the rings can contain one or more double bonds, but none of the rings has a completely conjugated π-electron system. The spiro heterocyclyl is preferably 6 to 14 membered spiro heterocyclyl, and more preferably 7 to 10 membered spiro heterocyclyl. According to the number of the spiro atoms shared between the rings, the spiro heterocyclyl can be divided into mono-spiro heterocyclyl, di-spiro heterocyclyl, or poly-spiro heterocyclyl, and the spiro heterocyclyl is preferably mono-spiro heterocyclyl or di-spiro heterocyclyl, and more preferably 3-membered/6-membered, 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

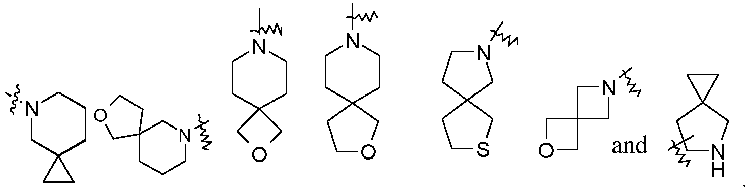

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The fused heterocyclyl is preferably 6 to 14 membered fused heterocyclyl, and more preferably 7 to 10 membered fused heterocyclyl. According to the number of membered rings, the fused heterocyclyl can be divided into bicyclic, tricyclic, tetracyclic or polycyclic fused heterocyclyl, and the fused heterocyclyl is preferably bicyclic or tricyclic fused heterocyclyl, and more preferably 5-membered/5-membered or 5-membered/6-membered bicyclic fused heterocyclyl. Non-limiting examples of fused heterocyclyl include:

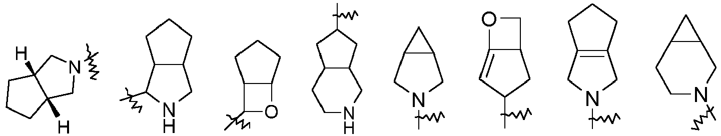

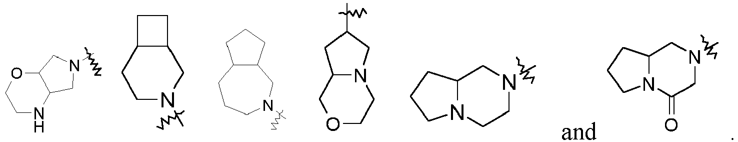

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The bridged heterocyclyl is preferably 6 to 14 membered bridged heterocyclyl, and more preferably 7 to 10 membered bridged heterocyclyl. According to the number of membered rings, the bridged heterocyclyl can be divided into bicyclic, tricyclic, tetracyclic or polycyclic bridged heterocyclyl, and the bridged heterocyclyl is preferably bicyclic, tricyclic or tetracyclic bridged heterocyclyl, and more preferably bicyclic or tricyclic bridged heterocyclyl. Non-limiting examples of bridged heterocyclyl include:

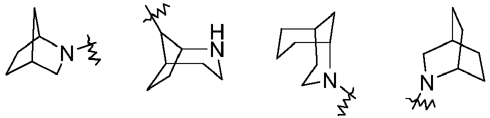

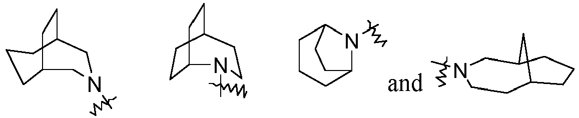

The heterocyclyl ring can be fused to the ring of aryl, heteroaryl or cycloalkyl, wherein the ring bound to the parent structure is heterocyclyl. Non-limiting examples thereof include: 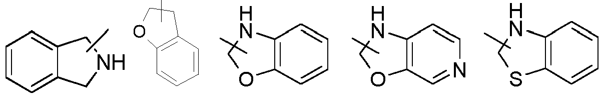 and the like.

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered all-carbon monocyclic ring or polycyclic fused ring (i.e. each ring in the system shares an adjacent pair of carbon atoms with another ring in the system) having a conjugated π-electron system, preferably 6 to 10 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. Non-limiting examples thereof include: 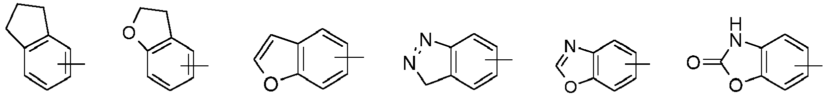 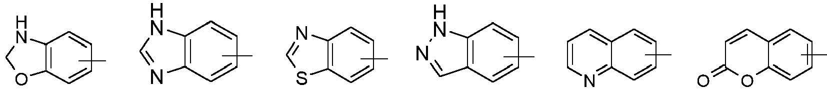 and

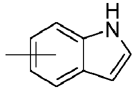

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of O, S and N. The heteroaryl is preferably 5 to 10 membered heteroaryl having 1 to 3 heteroatoms, more preferably 5 or 6 membered heteroaryl having 1 to 2 heteroatoms; preferably for example, imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, tetrazolyl, pyridyl, thienyl, pyrazolyl, pyrimidinyl, thiazolyl, and more preferably pyridyl. The heteroaryl ring can be fused to the ring of aryl, heterocyclyl or cycloalkyl, wherein the ring bound to the parent structure is heteroaryl ring. Non-limiting examples thereof include:

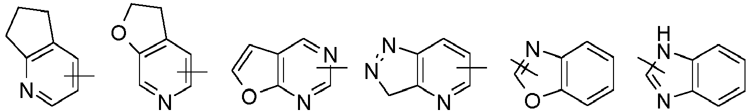

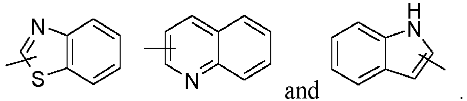

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "haloalkyl" refers to an alkyl group substituted by one or more halogens, wherein the alkyl is as defined above.

The term "haloalkoxy" refers to an alkoxy group substituted by one or more halogens, wherein the alkoxy is as defined above.

The term "hydroxyalkyl" refers to an alkyl group substituted by hydroxy(s), wherein the alkyl is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "oxo" refers to a =O group.

The term "carbonyl" refers to a C=O group.

The term "carboxy" refers to a -C(O)OH group.

The term "alkoxycarbonyl" refers to a -C(O)O(alkyl) or -C(O)O(cycloalkyl) group, wherein the alkyl and cycloalkyl are as defined above.

The term "acyl halide" refers to a compound containing a -C(O)-halogen group.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without paying excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present invention, which is safe and effective in mammals and has the desired biological activity.

### DESCRIPTION OF THE DRAWINGS

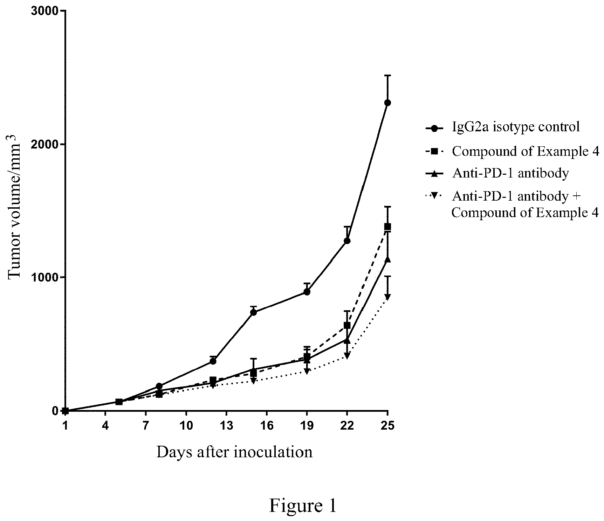

Figure 1 shows the effect of the compound of Example 4 administrated alone or in combination with an anti-mouse-PD-1 antibody on MC38 colorectal tumor growth in C57BL/6 mice.

### DETAILED DESCRIPTION OF THE INVENTION

The present invention will be further described with reference to the following examples, but the examples should not be considered as limiting the scope of the present invention.

### EXAMPLES

The structures of the compounds were identified by nuclear magnetic resonance (NMR) and/or mass spectrometry (MS). NMR shifts (δ) are given in 10⁻⁶ (ppm). NMR was determined by a Bruker AVANCE-400 machine. The solvents for determination were deuterated-dimethyl sulfoxide (DMSO-*d₆*), deuterated-chloroform (CDCl₃) and deuterated-methanol (CD₃OD), and the internal standard was tetramethylsilane (TMS).

MS was determined by a FINNIGAN LCQAd (ESI) mass spectrometer (manufacturer: Thermo, type: Finnigan LCQ advantage MAX).

High performance liquid chromatography (HPLC) was determined on an Agilent 1200DAD high pressure liquid chromatograph (Sunfire C18 150×4.6mm chromatographic column) and Waters 2695-2996 high pressure liquid chromatograph (Gimini C18 150×4.6mm chromatographic column).

Chiral HPLC analysis was determined on a LC-10A vp (Shimadzu) or SFC-analytical (Berger Instruments Inc.).

Yantai Huanghai HSGF254 or Qingdao GF254 silica gel plate was used as the thin-layer silica gel chromatography (TLC) plate. The dimension of the silica gel plate used in TLC was 0.15 mm to 0.2 mm, and the dimension of the silica gel plate used in product purification was 0.4 mm to 0.5 mm.

Yantai Huanghai 200 to 300 mesh silica gel was generally used as a carrier for column chromatography.

Prep Star SD-1 (Varian Instruments Inc.) or SFC-multigram (Berger Instruments Inc.) was used for chiral preparative column chromatography.

CombiFlash rapid preparation instrument used was Combiflash Rf200 (TELEDYNE ISCO).

The average kinase inhibition rates and IC₅₀ values were determined by a NovoStar ELISA (BMG Co., Germany).

The known starting materials of the present invention can be prepared by the known methods in the art, or can be purchased from ABCR GmbH & Co. KG, Acros Organnics, Aldrich Chemical Company, Accela ChemBio Inc., Dari chemical Company, or Shanghai Bide Pharmatech Ltd. etc.

Unless otherwise stated, the reactions were carried out under argon atmosphere or nitrogen atmosphere.

"Argon atmosphere" or "nitrogen atmosphere" means that a reaction flask is equipped with an argon or nitrogen balloon (about 1 L).

"Hydrogen atmosphere" means that a reaction flask is equipped with a hydrogen balloon (about 1 L).

Pressurized hydrogenation reactions were performed on a Parr 3916EKX hydrogenation instrument and a Qinglan QL-500 hydrogen generator or HC2-SS hydrogenation instrument.

In hydrogenation reactions, the reaction system was generally vacuumed and filled with hydrogen, and the above operation was repeated three times.

CEM Discover-S 908860 type microwave reactor was used in microwave reactions.

Unless otherwise stated, the solution refers to an aqueous solution.

Unless otherwise stated, the reaction temperature is room temperature from 20°C to 30°C.

The reaction process in the examples was monitored by thin layer chromatography (TLC). The developing solvent used in the reactions, the eluent system in column chromatography and the developing solvent system in thin layer chromatography for purification of the compounds included: A: dichloromethane/methanol system, and B: *n*-hexane/ethyl acetate system. The ratio of the volume of the solvent was adjusted according to the polarity of the compounds, and a small quantity of alkaline reagent such as triethylamine or acidic reagent such as acetic acid can also be added for adjustment.

### Example 1

### N-(1-(4-(Ethylsulfonyl)phenyl)-2-hydroxyethyl)-1-isopropyl-2-(2-(trifluoromethyl)benz yl)-1H-indole-5-carboxamide 1

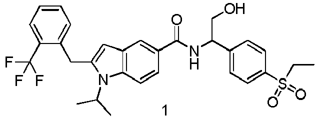

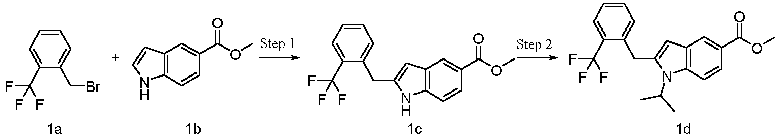

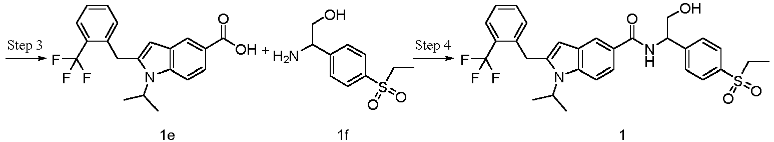

### Step 1

### Methyl 2-(2-(trifluoromethyl)benzyl)-1H-indole-5-carboxylate 1c

Methyl 1*H*-indole-5-carboxylate **1b** (400 mg, 2.29 mmol), 1-(bromomethyl)-2-(trifluoromethyl)benzene **1a** (574 mg, 2.4 mmol), bis(acetonitrile)palladium(II) chloride (118 mg, 0.46 mmol), bicyclo[2.2.1]-2-heptene (429 mg, 4.6 mmol) and sodium bicarbonate (384 mg, 4.6 mmol) were added to 10 mL of *N*,*N-*dimethylacetamide. The reaction solution was heated to 70°C and stirred for 16 hours under an argon atmosphere. After completion of the reaction, the reaction solution was poured into water, and extracted with ethyl acetate three times. The organic phases were combined, washed with water and saturated sodium chloride solution successively, dried over anhydrous sodium sulfate, and filtrated. The filtrate was concentrated under reduced pressure, and the resulting residue was purified by silica gel column chromatography with eluent system B to obtain the title compound **1c** (570 mg, yield: 74.9%).

### Step 2

### Methyl 1-isopropyl-2-(2-(trifluoromethyl)benzyl)-1H-indole-5-carboxylate 1d

Compound **1c** (500 mg, 1.5 mmol) was dissolved in 15 mL of *N*,*N*-dimethylformamide. The solution was added with 60% sodium hydride (120 mg, 3.0 mmol), and stirred at room temperature for 30 minutes. The reaction solution was then added with 2-iodopropane (1.02 g, 6.0 mmol), and reacted in a sealed reaction tube at 70°C for 16 hours. The reaction solution was cooled to room temperature, poured into water, and extracted with ethyl acetate three times. The organic phases were combined, washed with water once, dried over anhydrous sodium sulfate, and filtrated. The filtrate was concentrated under reduced pressure, and the resulting residue was purified by thin layer chromatography with developing solvent system B to obtain the title compound **1d** (80 mg, yield: 14.2%).

### Step 3

### 1-Isopropyl-2-(2-(trifluoromethyl)benzyl)-1H-indole-5-carboxylic acid 1e

Compound **1d** (80 mg, 0.21 mmol) was dissolved in a mixed solvent of 5 mL of methanol and 2 mL of tetrahydrofuran. The solution was added with 3 mL of 4*N* sodium hydroxide solution, and stirred at 60°C for 1 hour. The reaction solution was neutralized with concentrated hydrochloric acid, added with water, and extracted with ethyl acetate three times. The organic phases were combined, washed with saturated sodium chloride solution, dried over anhydrous sodium sulfate, and filtrated. The filtrate was concentrated under reduced pressure to obtain the crude title compound **1e** (70 mg), which was used directly in the next step without purification.

### Step 4

### N-(1-(4-(Ethylsulfonyl)phenyl)-2-hydroxyethyl)-1-isopropyl-2-(2-(trifluoromethyl)benz yl)-1H-indole-5-carboxamide 1

2-Amino-2-(4-ethylsulfonylphenyl)ethanol **1f** (67 mg, 0.29 mmol, prepared according to the method disclosed in the patent application "WO2016061160"), the crude compound **1e** (70 mg, 0.193 mmol), l-ethyl-(3-dimethylaminopropyl)carbonyldiimide hydrochloride (56 mg, 0.29 mmol), 1-hydroxybenzotriazole (40 mg, 0.29 mmol) and triethylamine (101 mg, 1 mmol) were added to 10 mL of dichloromethane. The reaction solution was stirred at room temperature for 16 hours. The reaction solution was concentrated under reduced pressure, and the resulting residue was purified by silica gel column chromatography with eluent system A to obtain the title compound **1** (40 mg, yield: 36.0%).
MS m/z (ESI): 573.5 [M+1].
¹H NMR (400HMz, CDCl₃) δ 8.12 (d, 1H), 7.92 (d, 2H), 7.76 (d, 1H), 7.69 (dd, 1H), 7.64 (d, 2H), 7.59 (d , 1H), 7.47-7.38 (m, 2H), 7.14-7.09 (m, 2H), 6.35 (s, 1H), 5.41-5.37 (m, 1H), 4.49-4.42 (m, 1H), 4.35 (s, 2H), 4.12-4.08 (m, 2H), 3.14 (q, 2H), 2.37 (brs, 1H), 1.52 (d, 6H), 1.32 (t, 3H).

### Example 2

### 2-(4-Chloro-2-(trifluoromethyl)benzyl)-N-(1-(4-(ethylsulfonyl)phenyl)-2-hydroxyethyl) -1-(2-fluoroethyl)-1H-indole-5-carboxamide 2

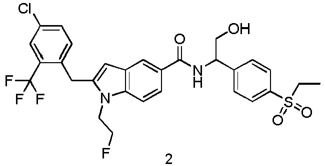

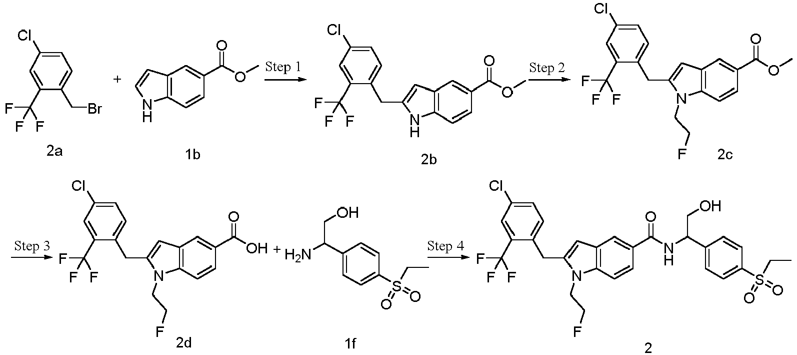

### Step 1

### Methyl 2-(4-chloro-2-(trifluoromethyl)benzyl)-1H-indole-5-carboxylate 2b

Compound **1b** (7 g, 39.96 mmol) and 1-(bromomethyl)-4-chloro-2-(trifluoromethyl)benzene **2a** (13.11 g, 47.95 mmol) were dissolved in 200 mL of *N*,*N-*dimethylacetamide. Then bis(acetonitrile)palladium(II) chloride (2.07 g, 7.99 mmol), bicyclo[2.2.1]-2-heptene (3.76 g, 39.96 mmol) and sodium carbonate (8.47 g, 79.92 mmol) were added. The reaction solution was heated to 80°C and stirred for 17 hours under an argon atmosphere. The reaction solution was cooled and filtrated. The filtrate was concentrated under reduced pressure, and the resulting residue was purified by silica gel column chromatography with eluent system B to obtain the title compound **2b** (13 g, yield: 88.47%).
MS m/z (ESI): 368.1 [M+1].

### Step 2

### Methyl 2-(4-chloro-2-(trifluoromethyl)benzyl)-1-(2-fluoroethyl)-1H-indole-5-carboxylate 2c

Compound **2b** (0.3 g, 815.77 µmol), 1-bromo-2-fluoroethane (310.7 mg, 2.45 mmol) was dissolved in 10 mL of *N*,*N*-dimethylformamide. The reaction solution was added with cesium carbonate (797.38 mg, 2.45 mmol), and reacted under a microwave condition at 100°C for one hour. The reaction solution was cooled and filtrated. The filtrate was concentrated under reduced pressure, and the resulting residue was purified by silica gel column chromatography with eluent system B to obtain the title compound **2c** (0.25 g, yield: 74.06%).
MS m/z (ESI): 414.1 [M+1].

### Step 3

### 2-(4-Chloro-2-(trifluoromethyl)benzyl)-1-(2-fluoroethyl)-1H-indole-5-carboxylic acid 2d

Compound **2c** (0.25 g, 604.17 µmol) was dissolved in 20 mL of methanol. The solution was added with 1.5 mL of 4N sodium hydroxide solution, and stirred under reflux for 1 hour. The reaction solution was cooled to room temperature, and added dropwise with 1*M* hydrochloric acid to adjust the pH to 3∼4. The reaction solution was added with 20 mL of water and 20 mL of ethyl acetate, and extracted with ethyl acetate (20mL×2). The organic phases were combined, dried over anhydrous sodium sulfate, and filtrated. The filtrate was concentrated under reduced pressure, and the resulting residue was purified by silica gel column chromatography with eluent system A to obtain the title compound **2d** (0.24 g, yield: 99.4%).
MS m/z (ESI): 400.1 [M+1].

### Step 4

### 2-(4-Chloro-2-(trifluoromethyl)benzyl)-N-(1-(4-(ethylsulfonyl)phenyl)-2-hydroxyethyl) -1-(2-fluoroethyl)-1H-indole-5-carboxamide 2

Compound **2d** (10 mg, 25.01 µmol) was dissolved in 2 mL of *N*,*N*-dimethylformamide. Compound **1f** (8.67 mg, 37.83 µmol) and *N*,*N*-diisopropylethylamine (6.47 mg, 50.03 µmοl) were added, followed by 2-(7-azobenzotriazole)-*N*,*N*,*N*',*N*'-tetramethyluronium hexafluorophosphate (11.77 mg, 50.03 µmol). The reaction solution was stirred at room temperature for 2 hours, concentrated under reduced pressure, and the resulting residue was purified by high performance liquid chromatography to obtain the title compound **2** (7.9 mg, yield: 51.7%).
MS m/z (ESI): 611.5 [M+1].
¹H NMR (400MHz, CDCl₃) δ 8.13 (s, 1H), 7.93-7.90 (m, 2H), 7.77-7.75 (m, 2H), 7.64-7.62 (m, 2H), 7.47-7.45 (m, 1H), 7.37-7.35 (m, 1H), 7.16-7.14 (m, 1H), 7.13-7.11 (m, 1H), 6.33 (s, 1H), 5.40-5.38 (m, 1H), 4.70-4.68 (m, 1H), 4.57-4.56 (m, 1H), 4.37-4.35 (m, 1H), 4.35 (s, 2H), 4.30-4.31 (m, 1H), 4.11-4.06 (m, 2H), 3.16-3.11 (m, 2H), 1.33-1.29 (m, 3H).

### Example 3, 4

### (S)-2-(4-Chloro-2-(trifluoromethyl)benzyl)-N-(1-(4-(ethylsulfonyl)phenyl)-2-hydroxyet hyl)-1-(2-fluoroethyl)-1H-indole-5-carboxamide 3

### (R)-2-(4-Chloro-2-(trifluoromethyl)benzyl)-N-(1-(4-(ethylsulfonyl)phenyl)-2-hydroxyet hyl)-1-(2-fluoroethyl)-1H-indole-5-carboxamide 4

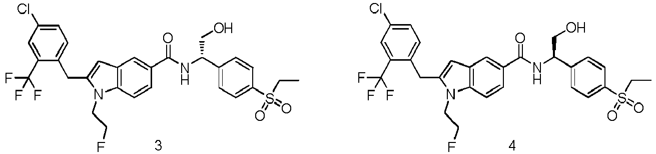

Compound **2** (120 mg, 0.197 mmol) was separated chirally (separation conditions: Superchiral S-AD (Chiralway), 2cm I.D. × 25 cm Length, 5 µm, mobile phase: carbon dioxide/ethanol/diethylamine = 60/40/0.05 (v/v/v), flow rate: 50 g/min). The corresponding fractions were collected and concentrated under reduced pressure to obtain the title compound **3** (52 mg) and title compound **4** (52 mg).

Compound 3:
MS m/z (ESI): 610.9 [M+1].

Chiral HPLC analysis: retention time 7.882 minutes, chiral purity: 100% (chromatographic column: Lux Amylose-1 (AD) 4.6×150mm 5 µm (equipped with a guard column); mobile phase: *n*-hexane/ethanol (containing 0.1% of diethylamine) = 60/40 (v/v)).
¹H NMR (400MHz, CDCl₃) δ 8.13 (s, 1H), 7.93-7.90 (m, 2H), 7.77-7.75 (m, 2H), 7.64-7.62 (m, 2H), 7.47-7.45 (m, 1H), 7.37-7.35 (m, 1H), 7.16-7.14 (m, 1H), 7.13-7.11 (m, 1H), 6.33 (s, 1H), 5.40-5.38 (m, 1H), 4.70-4.68 (m, 1H), 4.57-4.56 (m, 1H), 4.37-4.35 (m, 1H), 4.35 (s, 2H), 4.31-4.30 (m, 1H), 4.11-4.06 (m, 2H), 3.16-3.11 (m, 2H), 1.33-1.29 (m, 3H).

Compound **4**:
MS m/z (ESI): 611.0 [M+1].

Chiral HPLC analysis: retention time 11.747 minutes, chiral purity: 100% (chromatographic column: Lux Amylose-1 (AD) 4.6×150mm 5 µm (equipped with a guard column); mobile phase: *n*-hexane/ethanol (containing 0.1% of diethylamine) = 60/40 (v/v)).
¹H NMR (400MHz, CDCl₃) δ 8.12 (s, 1H), 7.93-7.91 (m, 2H), 7.76-7.74 (m, 2H), 7.65-7.63 (m, 2H), 7.47-7.45 (m, 1H), 7.37-7.35 (m, 1H), 7.15-7.11 (m, 2H), 6.33 (s, 1H), 5.39-5.38 (m, 1H), 4.70-4.69 (m, 1H), 4.59-4.56 (m, 1H), 4.37-4.36 (m, 1H), 4.35 (s, 2H), 4.31-4.30 (m, 1H), 4.11-4.06 (m, 2H), 3.17-3.11 (m, 2H), 2.33 (brs, 1H), 1.34-1.30 (m, 3H).

### Example 5

### (R)-2-(4-Chloro-2-(trifluoromethyl)benzyl)-N-(1-(4-(ethylsulfonyl)phenyl)-2-hydroxyet hyl)-6-fluoro-1-(2-fluoroethyl)-1H-indole-5-carboxamide 5

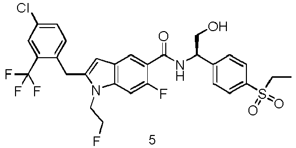

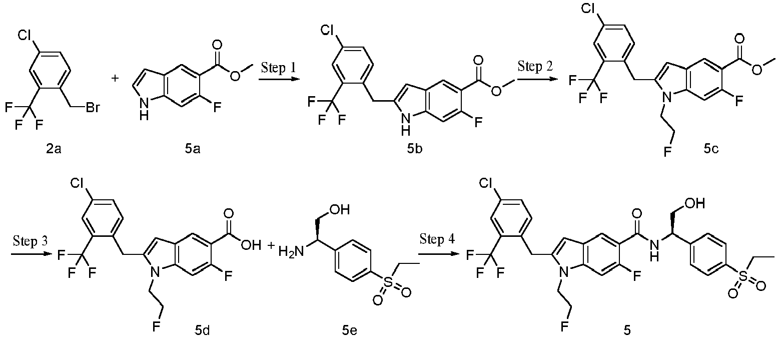

### Step 1

### Methyl 2-(4-chloro-2-(trifluoromethyl)benzyl)-6-fluoro-1H-indole-5-carboxylate 5b

Methyl 6-fluoro-1*H*-indole-5-carboxylate **5a** (500 mg, 2.59 mmol), compound 2a (1.06 g, 88 mmol), bis(acetonitrile)palladium(II) chloride (67.15 mg, 258.83 µmol), bicyclo[2.2.1]-2-heptene (487.40 mg, 5.18 mmol) and potassium carbonate (714.38 mg, 5.18 mmol) were added to 20 mL of *N*,*N-*dimethylacetamide. The reaction solution was heated to 80°C and stirred for 16 hours under an argon atmosphere. The reaction solution was cooled to room temperature and filtrated. The filtrate was poured into water, and extracted with ethyl acetate. The organic phases were combined, dried over anhydrous sodium sulfate, and filtrated. The filtrate was concentrated under reduced pressure, and the resulting residue was purified by silica gel column chromatography with eluent system B to obtain the title compound **5b** (600 mg, yield: 60.1%).
MS m/z (ESI): 386.1 [M+1].

### Step 2

### Methyl 2-(4-chloro-2-(trifluoromethyl)benzyl)-6-fluoro-1-(2-fluoroethyl)-1H-indole-5-carboxyl ate 5c

Compound **5b** (100 mg, 259.24 µmol), 1-fluoro-2-iodo-ethane (67.64 mg, 388.86 µmol) and cesium carbonate (254.32 mg, 777.72 µmοl) were added to 5 mL of acetonitrile. The reaction solution was reacted under a microwave condition at 100°C for one hour. The reaction solution was poured into water, and extracted with ethyl acetate. The organic phases were combined, dried over anhydrous sodium sulfate, and filtrated. The filtrate was concentrated under reduced pressure, and the resulting residue was purified by silica gel column chromatography with eluent system B to obtain the title compound **5c** (80 mg, yield: 71.5%).
MS m/z (ESI): 432.1 [M+1].

### Step 3

### 2-(4-Chloro-2-(trifluoromethyl)benzyl)-6-fluoro-1-(2-fluoroethyl)-1H-indole-5-carboxy lie acid 5d

Compound **5c** (80 mg, 185.28 µmol) and sodium hydroxide (37.05 mg, 926.39 µmοl) were added to a mixed solvent of 6 mL of methanol and 0.5 mL of water. The reaction solution was stirred at 60°C for 2 hours. Methanol was removed under reduced pressure, and the resulting residue was added dropwise with 1*M* diluted hydrochloric acid to adjust the pH to ∼3. The reaction solution was extracted with ethyl acetate, and the organic phase was dried over anhydrous sodium sulfate, and filtrated. The filtrate was concentrated under reduced pressure to obtain the crude title compound **5d** (50 mg), which was used directly in the next step without purification.
MS m/z (ESI): 418.0 [M+1].

### Step 4

### (R)-2-(4-Chloro-2-(trifluoromethyl)benzyl)-N-(1-(4-(ethylsulfonyl)phenyl)-2-hydroxyet hyl)-6-fluoro-1-(2-fluoroethyl)-1H-indole-5-carboxamide 5

(*R*)-2-Amino-2-(4-(ethylsulfonyl)phenyl)ethanol **5e** (16.47 mg, 71.81 µmol, prepared according to the method disclosed in the patent application "WO2016061160"), the crude compound **5d** (30 mg, 71.81 µmol), 1-ethyl-(3-dimethylaminopropyl)carbonyldiimide hydrochloride (20.57 mg, 107.72 µmοl), 1-hydroxybenzotriazole (16.39 mg, 107.72 µmol) and *N*,*N*-diisopropylethylamine (27.84 mg, 215.43 µmol) were added to 3 mL of *N*,*N*-dimethylformamide. The reaction solution was stirred at room temperature for 16 hours. The reaction solution was extracted with ethyl acetate, and the organic phases were combined, dried over anhydrous sodium sulfate, and filtrated. The filtrate was concentrated under reduced pressure, and the resulting residue was purified by silica gel column chromatography with eluent system B to obtain the title compound **5** (19 mg, yield: 42.1%).
MS m/z (ESI): 629.5 [M+1].
¹H NMR (400MHz, CDCl₃) δ 8.26 (d, 1H), 7.89 (d, 2H), 7.72-7.60 (m, 4H), 7.42 (d, 1H), 7.11-7.03 (m, 2H), 6.27 (s, 1H), 5.41-5.39 (m, 1H), 4.63-4.51 (m, 2H), 4.27-4.19 (m, 4H), 4.06-4.01 (m, 2H), 3.12-3.07 (m, 2H), 2.35 (brs, 1H), 1.30-1.27 (m, 3H).

### Example 6

### (R)-2-(4-Chloro-2-(trifluoromethyl)benzyl)-1-cyclopropyl-N-(1-(4-(ethylsulfonyl)pheny l)-2-hydroxyethyl)-6-fluoro-1H-indole-5-carboxamide 6

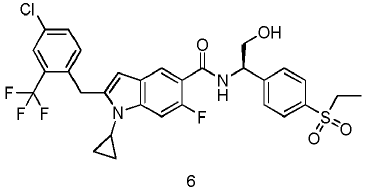

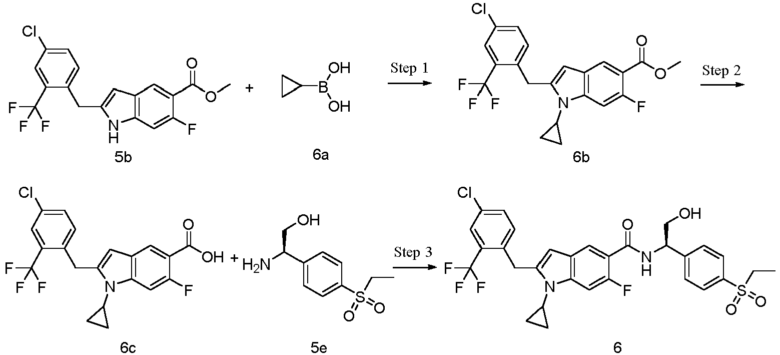

### Step 1

### Methyl 2-(4-chloro-2-(trifluoromethyl)benzyl)-1-cyclopropyl-6-fluoro-1H-indole-5-carboxylate 6b

Compound **5b** (100 mg, 259.24 µmol), cyclopropylboronic acid **6a** (44.54 mg, 518.48 µmol), 2,2'-bipyridine (48.59 mg, 311.09 µmol), copper acetate (56.50 mg, 311.09 µmol) and sodium carbonate (54.95 mg, 518.48 µmol) were added to 20 mL of tetrahydrofuran. The reaction solution was stirred at 60°C for 16 hours under an argon atmosphere. The reaction solution was filtrated, and the filtrate was extracted with ethyl acetate, dried over anhydrous sodium sulfate, and filtrated. The filtrate was concentrated under reduced pressure, and the resulting residue was purified by silica gel column chromatography with eluent system B to obtain the title compound **6b** (80 mg, yield: 72.47%).
MS m/z (ESI): 426.1 [M+1].

### Step 2

### 2-(4-Chloro-2-(trifluoromethyl)benzyl)-1-cyclopropyl-6-fluoro-1H-indole-5-carboxylic acid 6c

Compound **6b** (90 mg, 211.37 µmol) and sodium hydroxide (42.27 mg, 1.06 mmol) were added to a mixed solvent of 6 mL of methanol and 0.5 mL of water. The solution was stirred at 60°C for 2 hours. TMethanol was removed under reduced pressure, and the resulting residue was added dropwise with 1*M* hydrochloric acid to adjust the pH to ∼3. The solution was extracted with ethyl acetate, and the organic phase was dried over anhydrous sodium sulfate, and filtrated. The filtrate was concentrated under reduced pressure to obtain the crude title compound **6c** (60 mg), which was used directly in the next step without purification.
MS m/z (ESI):412.0 [M+1].

### Step 3

### (R)-2-(4-Chloro-2-(trifluoromethyl)benzyl)-1-cyclopropyl-N-(1-(4-(ethylsulfonyl)pheny 1)-2-hydroxyethyl)-6-fluoro-1H-indole-5-carboxamide 6

Compound **5e** (16.71 mg, 72.86 µmol), the crude compound **6c** (30 mg, 72.86 µmοl), 1-ethyl-(3-dimethylaminopropyl)carbonyldiimide hydrochloride (20.87 mg, 109.28 µmol), 1-hydroxybenzotriazole (16.63 mg, 109.28 µmol) and *N*,*N*-diisopropylethylamine (28.25 mg, 218.57 µmol) were added to 3 mL of *N*,*N*-dimethylformamide. The reaction solution was stirred at room temperature for 16 hours. The reaction solution was extracted with ethyl acetate, and the organic phases were combined, dried over anhydrous sodium sulfate, and filtrated. The filtrate was concentrated under reduced pressure, and the resulting residue was purified by silica gel column chromatography with eluent system B to obtain the title compound **6** (15 mg, yield: 33.0%).
MS m/z (ESI): 623.5 [M+1].
¹H NMR (400MHz, CDCl₃) δ 8.23 (d, 1H), 7.91 (d, 2H), 7.71 (d, 2H), 7.62 (d, 2H), 7.42 (d, 1H), 7.28 (d, 1H), 7.03 (d, 1H), 6.17 (s, 1H), 5.40 (s , 1H), 4.37 (s , 2H), 4.08-4.04 (m, 2H), 3.14-3.10 (m, 2H), 2.98-2.90 (m, 1H), 2.21 (brs, 1H), 1.31-1.27 (m, 3H), 1.14-1.12 (m, 2H), 0.98-0.89 (m, 2H).

### Example 7

### N-((R)-1-(4-(Ethylsulfonyl)phenyl)-2-hydroxyethyl)-1-(2-fluoroethyl)-2-((3-(trifluorom ethyl)morpholino)methyl)-1H-indole-5-carboxamide 7

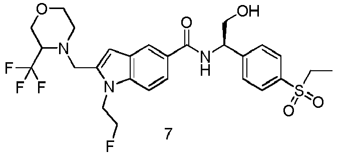

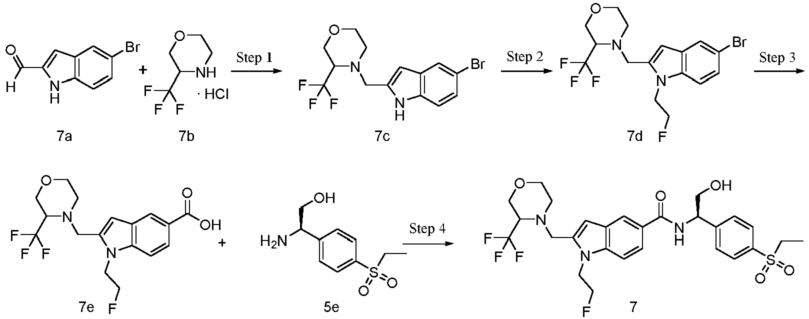

### Step 1

### 4-((5-Bromo-1H-indol-2-yl)methyl)-3-(trifluoromethyl)morpholine 7c

5-Bromo-1*H*-indole-2-carbaldehyde **7a** (120 mg, 0.54 mmol, prepared according to the known method disclosed in "Journal of Medicinal Chemistry, 2014, 57(2), 364-377") was dissolved in 10 mL of 1,2-dichloroethane. The solution was added with 3-(trifluoromethyl)morpholine hydrochloride **7b** (120 mg, 0.63 mmol) and 5 drops of acetic acid, and stirred for 1.5 hours. The reaction solution was added with sodium triacetylborohydride (240 mg, 1.1 mmol), and stirred for 16 hours. The reaction solution was added with saturated sodium bicarbonate solution, and extracted with ethyl acetate three times. The organic phases were combined, washed with saturated sodium chloride solution, dried over anhydrous sodium sulfate, and filtrated. The filtrate was concentrated under reduced pressure, and purified by CombiFlash rapid preparation instrument with eluent system B to obtain the title compound **7c** (150 mg, yield: 76.5%).
MS m/z (ESI): 363 [M+1].

### Step 2

### 4-((5-Bromo-1-(2-fluoroethyl)-1H-indol-2-yl)methyl)-3-(trifluoromethyl)morpholine 7d

Compound **7c** (80 mg, 0.22 mmol), 1-fluoro-2-iodoethane (60 µL), cesium carbonate (200 mg, 0.61 mmol) and 10 mL of *N*,*N*-dimethylformamide were added to a microwave reaction tube. The reaction solution was reacted under a microwave condition at 100°C for one hour. The reaction solution was added with water, and extracted with ethyl acetate. The organic phases were combined, dried over anhydrous sodium sulfate, and filtrated. The filtrate was concentrated under reduced pressure, and the resulting residue was purified by CombiFlash rapid preparation instrument with eluent system B to obtain the title compound **7d** (92 mg, yield: 100%).
MS m/z (ESI): 409 [M+1].

### Step 3

### 1-(2-Fluoroethyl)-2-((3-(trifluoromethyl)morpholino)methyl)-1H-indole-5-carboxylic acid 7e

Compound **7d** (28 mg, 68 µmol), molybdenum hexacarbonyl (35 mg, 133 µmol), *trans*-bis(acetato)bis[o-(di-o-tolyphosphino)benzyl]dipalladium(II) (14 mg, 15 µmοl), tri-*tert*-butylphosphine tetrafluoroborate (14 mg, 48 µmol), 1,8-diazabicycloundec-7-ene (50 µL) were added to a mixed solvent of water (50 µL) and 1,4-dioxane (0.5 mL). The reaction solution was reacted under a microwave condition at 150°C for 15 minutes. The reaction solution was purified by CombiFlash rapid preparation instrument with eluent system A to obtain the title compound **7e** (15 mg, yield: 58%).
MS m/z (ESI): 375 [M+1].

### Step 4

### N-((R)-1-(4-(Ethylsulfonyl)phenyl)-2-hydroxyethyl)-1-(2-fluoroethyl)-2-((3-(trifluorom ethyl)morpholino)methyl)-1H-indole-5-carboxamide 7

Compound **7e** (18 mg, 48 µmol) was dissolved in 0.8 mL of *N*,*N*-dimethylformamide, then compound **5e** (12 mg, 52 µmol), *N*,*N*-diisopropylethylamine (40 µL) and 2-(7-azobenzotriazole)-*N*,*N*,*N*',*N*'-tetramethyluronium hexafluorophosphate (30 mg, 78 µmol) were added. The reaction solution was stirred for 2 hours, and then purified by high performance liquid chromatography to obtain the title compound **7** (6 mg, yield: 21.4%).
MS m/z (ESI): 586 [M+1].
¹H NMR (400MHz, CDCl₃) δ 8.03 (s, 1H), 7.79 (d, 2H), 7.64 (d, 1H), 7.51 (d, 2H), 7.30 (d, 1H), 7.11 (d, 1H), 6.47 (s, 1H), 5.24 (brs, 1H), 4.75-4.73 (m, 1H), 4.62-4.37 (m, 3H), 4.12 (d, 1H), 4.05-3.81 (m, 4H), 3.81-3.51 (m, 3H), 3.08-2.87 (m, 4H), 2.38 (d, 1H), 1.20 (t, 3H).

### Example 8

### (R)-1-Cyclopropyl-N-(1-(4-(ethylsulfonyl)phenyl)-2-hydroxyethyl)-6-fluoro-2-((3-meth yl-5-(trifluoromethyl)-1H-pyrazol-1-yl)methyl)-1H-indole-5-carboxamide 8

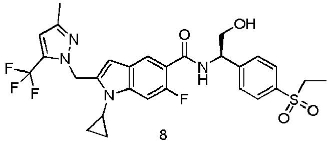

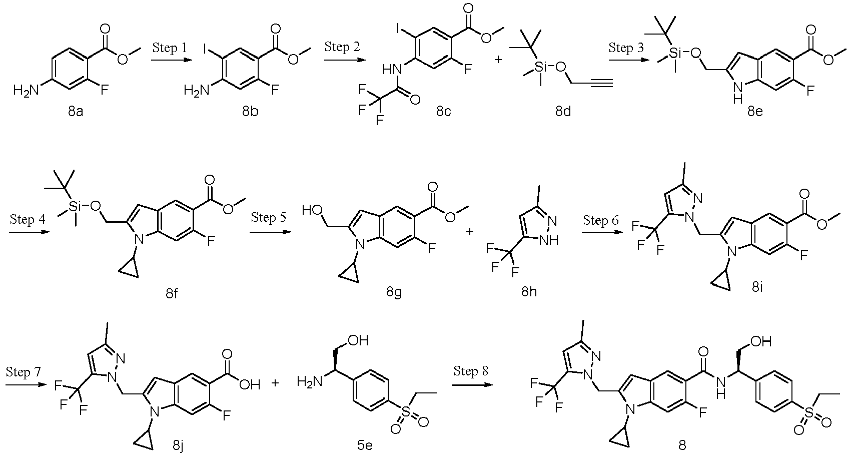

### Step 1

### Methyl 4-amino-2-fluoro-5-iodobenzoate 8b

Methyl 4-amino-2-fluorobenzoate (3.4 g, 20.2 mmol, prepared according to the method disclosed in the patent application "WO2013068467") was dissolved in 30 mL of a mixed solvent of dichloromethane and methanol (V:V=2:1). The solution was added with iodine chloride (3.6 g, 22.2 mmol) and calcium carbonate (4.03 g, 40.4 mmol) at room temperature, and stirred for 2 hours. The reaction solution was filtrated. The filtrate was washed with saturated sodium thiosulfate solution (50 mL×1), dried over anhydrous sodium sulfate, and filtrated. The filtrate was concentrated under reduced pressure, and the resulting residue was purified by silica gel column chromatography with eluent system B to obtain the title compound **8b** (6.5 g, yield: 100%).
MS m/z (ESI): 296 [M+1].

### Step 2

### Methyl 2-fluoro-5-iodo-4-(2,2,2-trifluoroacetamido)benzoate 8c

Compound **8b** (6.5 g, 20.2 mmol) was dissolved in 100 mL of dichloromethane. The solution was added with trifluoroacetic anhydride (3.6 g, 22.2 mmol) in an ice bath, and stirred for 1 hour. The reaction solution was concentrated under reduced pressure, and the resulting residue was purified by silica gel column chromatography with eluent system B to obtain the title compound **8c** (5.4 g, yield: 69%).
MS m/z (ESI): 392 [M+1].

### Step 3

### Methyl 2-(((tert-butyl-dimethyl silyl)oxy)methyl)-6-fluoro-1H-indole-5-carboxylate 8e

Compound **8c** (2.28 g, 5.8 mmol) and *tert*-butyldimethyl(2-propyn-1-yloxy)silane **8d** (1.49 g, 8.74 mmol, prepared according to the known method disclosed in "Journal of the American Chemical Society, 2016, 138(24), 7532-7535") were dissolved in 10 mL of *N*,*N*-dimethylformamide. The solution was added with bis(triphenylphosphine)palladium chloride (0.61 g, 0.88 mmol), cuprous iodide (0.222 g, 1.17 mmol) and triethylamine (4.07 mL, 29.2 mmol), and stirred at 70°C for 4 hours. The reaction solution was added with 40 mL of water, and extracted with ethyl acetate (30 mL×3). The organic phases were combined, washed with saturated sodium chloride solution (30 mL×1), dried over anhydrous sodium sulfate, and filtrated. The filtrate was concentrated under reduced pressure, and the resulting residue was purified by silica gel column chromatography with eluent system B to obtain the title compound **8e** (1.29 g, yield: 65%).
MS m/z (ESI): 338 [M+1].

### Step 4

### Methyl 2-(((tert-butyldimethylsilyl)oxy)methyl)-1-cyclopropyl-6-fluoro-1H-indole-5-carboxyla te 8f

Compound **8e** (0.633 g, 1.88 mmol) and cyclopropylboronic acid (1.61 g, 18.8 mmol) were dissolved in 5 mL of 1,2-dichloroethane. The solution was added with copper acetate (1.43 g, 7.88 mmol), 2,2-bipyridine (1.23 g, 7.88 mmol) and sodium carbonate (0.84 g, 7.88 mmol), and stirred at 80°C for 16 hours. The reaction solution was filtrated, and the filter cake was washed with dichloromethane. The filtrate was concentrated under reduced pressure, and the resulting residue was purified by silica gel column chromatography with eluent system B to obtain the title compound **8f** (350 mg, yield: 50%).
MS m/z (ESI): 378 [M+1].

### Step 5

### Methyl 1-cyclopropyl-6-fluoro-2-(hydroxymethyl)-1H-indole-5-carboxylate 8g

Compound **8f** (350 mg, 0.927 mmol) was dissolved in 10 mL of tetrahydrofuran. The solution was added with a solution of tetrabutylammonium fluoride in tetrahydrofuran (1.85 mL, 1.85 mmol) in an ice bath, and stirred at 0°C for 2 hours. The reaction solution was added with 10 mL of water, and extracted with ethyl acetate (10 mL×3). The organic phases were combined, washed with saturated sodium chloride solution (10 mL×1), dried over anhydrous sodium sulfate, and filtrated. The filtrate was concentrated under reduced pressure, and the resulting residue was purified by silica gel column chromatography with eluent system B to obtain the title compound **8g** (230 mg, yield: 68%).
MS m/z (ESI): 264 [M+1].

### Step 6

### Methyl 1-cyclopropyl-6-fluoro-2-((3-methyl-5-(trifluoromethyl)-1H-pyrazol-1-yl)methyl)-1H-i ndole-5-carboxylate 8i

Compound **8g** (84 mg, 0.32 mmol) and 3-methyl-5-trifluoromethylpyrazole **8h** (192 mg, 1.28 mmol, prepared according to the known method disclosed in "Tetrahedron Letters, 2016, 57(14), 1555-1559") were dissolved in 10 mL of tetrahydrofuran. The solution was added with triphenylphosphine (336 mg, 1.85 mmol) and diethyl azodicarboxylate (200 µL, 1.28 mmol) at room temperature, and stirred for 16 hours. The reaction solution was added with 30 mL of ethyl acetate, washed with water (10 mL) and saturated sodium chloride solution (10 mL) successively, dried over anhydrous sodium sulfate, and filtrated. The filtrate was concentrated under reduced pressure, and the resulting residue was purified by silica gel column chromatography with eluent system B to obtain the title compound **8i** (49 mg, yield: 39%).
MS m/z (ESI): 396 [M+1].

### Step 7

### 1-Cyclopropyl-6-fluoro-2-((3-methyl-5-(trifluoromethyl)-1H-pyrazol-1-yl)methyl)-1H-i ndole-5-carboxylic acid 8j

Compound **8i** (50 mg, 0.124 mmol) was dissolved in 1 mL of methanol. The solution was added with 2 *M* potassium hydroxide solution (1 mL, 2 mmol) at room temperature, and stirred at room temperature for 16 hours. The reaction solution was added with 6 *M* hydrochloric acid to adjust the pH to 1∼2, added with 10 mL of water, and extracted with ethyl acetate (10 mL×3). The organic phases were combined, washed with saturated sodium chloride solution (10 mL×1), dried over anhydrous sodium sulfate, and filtrated. The filtrate was concentrated under reduced pressure to obtain the crude title compound **8j** (50 mg), which was used directly in the next step without purification.
MS m/z (ESI): 382 [M+1].

### Step 8

### (R)-1-Cyclopropyl-N-(1-(4-(ethylsulfonyl)phenyl)-2-hydroxyethyl)-6-fluoro-2-((3-meth yl-5-(trifluoromethyl)-1H-pyrazol-1-yl)methyl)-1H-indole-5-carboxamide 8

Compound **8j** (3.9 mg, 0.01 mmol) and compound **5e** (4 mg, 0.015 mmol) were dissolved in 1 mL of dichloromethane. The solution was added with 1-ethyl-(3-dimethylaminopropyl)carbodiimide hydrochloride (3.9 mg, 0.02 mmol), 1-hydroxybenzotriazole (2.7 mg, 0.02 mmol) and triethylamine (7 µL, 0.05 mmol) at room temperature, and stirred at room temperature for 4 hours. The reaction solution was concentrated under reduced pressure, and the resulting residue was purified by high performance liquid chromatography to obtain the title compound **8** (3 mg, yield: 50%).
MS m/z (ESI): 593 [M+1].

### Example 9

### (R)-N-(1-(4-(Ethylsulfonyl)phenyl)-2-hydroxyethyl)-6-fluoro-1-(2-fluoroethyl)-2-((3-m ethyl-5-(trifluoromethyl)-1H-pyrazol-1-yl)methyl)-1H-indole-5-carboxamide 9

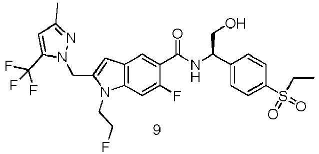

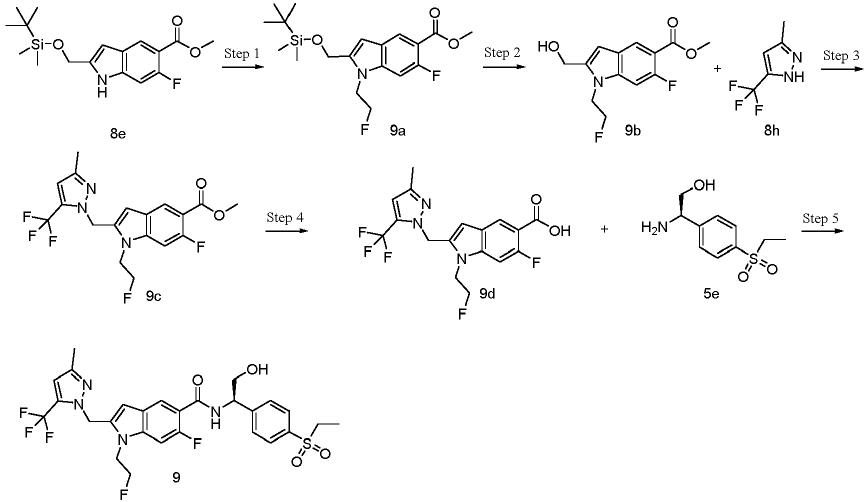

### Step 1

### Methyl 2-(((tert-butyldimethylsilyl)oxy)methyl)-6-fluoro-1-(2-fluoroethyl)-1H-indole-5-carbox ylate 9a

Compound **8e** (0.6 g, 1.78 mmol) was dissolved in 5 mL of *N*,*N*-dimethylformamide. The solution was added with 2-fluoroiodoethane (0.775 g, 4.45 mmol) and potassium carbonate (0.862 g, 6.24 mmol), and stirred at 80°C for 4 hours. The reaction solution was added with 40 mL of water, and extracted with ethyl acetate (20 mL×3). The organic phases were combined, washed with saturated sodium chloride solution (30 mL×1), dried over anhydrous sodium sulfate, and filtrated. The filtrate was concentrated under reduced pressure, and the resulting residue was purified by silica gel column chromatography with eluent system B to obtain the title compound **9a** (610 mg, yield: 90%).
MS m/z (ESI): 384 [M+1].

### Step 2

### Methyl 6-fluoro-1-(2-fluoroethyl)-2-(hydroxymethyl)-1H-indole-5-carboxylate 9b

In accordance with the synthetic route of compound **8g** in Step 5 of Example 8, the starting compound **8f** was replaced with compound **9a**, accordingly, the title compound **9b** (340 mg, yield: 80%) was prepared.
MS m/z (ESI): 270 [M+1].

### Step 3

### Methyl 6-fluoro-1-(2-fluoroethyl)-2-((3-methyl-5-(trifluoromethyl)-1H-pyrazol-1-yl)methyl)-1 H-indole-5-carboxylate 9c

In accordance with the synthetic route of compound **8i** in Step 6 of Example 8, the starting compound **8g** was replaced with compound **9b**, accordingly, the title compound **9c** (170 mg, yield: 73%) was prepared.
MS m/z (ESI): 402 [M+1].

### Step 4

### 6-Fluoro-1-(2-fluoroethyl)-2-((3-methyl-5-(trifluoromethyl)-1H-pyrazol-1-yl)methyl)-1 H-indole-5-carboxylic acid 9d

In accordance with the synthetic route of compound **8j** in Step 7 of Example 8, the starting compound **8i** was replaced with compound **9c**, accordingly, the crude title compound **9d** (150 mg, yield: 90%) was prepared, which was used directly in the next step without purification.
MS m/z (ESI): 388 [M+1].

### Step 5

### (R)-N-(1-(4-(Ethylsulfonyl)phenyl)-2-hydroxyethyl)-6-fluoro-1-(2-fluoroethyl)-2-((3-m ethyl-5-(trifluoroethyl)-1H-pyrazol-1-yl)methyl)-1H-indole-5-carboxamide 9

In accordance with the synthetic route of compound **8** in Step 8 of Example 8, the starting compound **8j** was replaced with compound **9d**, accordingly, the title compound **9** (1.4 mg, yield: 20%) was prepared.
MS m/z (ESI): 599 [M+1].

### Example 10

### (R)-2-(4-Chloro-2-(trifluoromethyl)benzyl)-N-(1-(4-(ethylsulfonyl)phenyl)-2-hydroxyet hyl)-1-isopropyl-1H-indole-5-carboxamide 10

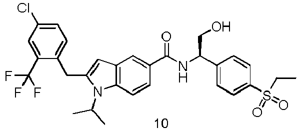

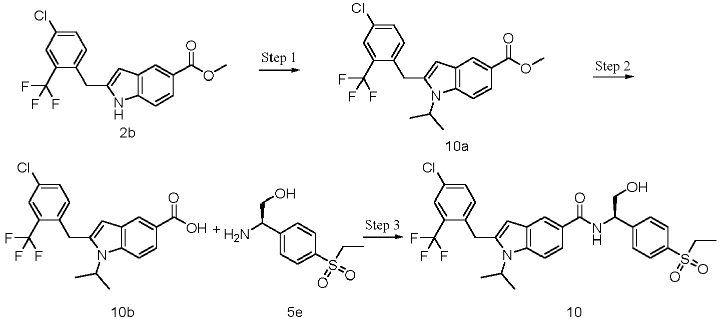

### Step 1

### Methyl 2-(4-chloro-2-(trifluoromethyl)benzyl)-1-isopropyl-1H-indole-5-carboxylate 10a

Compound **2b** (47.5 mg, 0.13 mmol) was dissolved in 1.5 mL of *N*,*N*-dimethylformamide. The solution was added with 60% sodium hydride (32 mg, 0.774 mmol), followed by 2-iodopropane (77.4 µL, 0.774 mmol), and reacted at 75°C for 16 hours. The reaction solution was cooled to room temperature, added with 15 mL of water, and extracted with ethyl acetate (10 mL×3). The organic phases were combined, washed with saturated sodium chloride solution (10 mL×3), dried over anhydrous sodium sulfate, and filtrated. The filtrate was concentrated under reduced pressure, and the resulting residue was purified by silica gel column chromatography with developing solvent system B to obtain the title compound **10a** (20 mg, yield: 40%).
MS m/z (ESI): 410 [M+1].

### Step 2

### 2-(4-Chloro-2-(trifluoromethyl)benzyl)-1-isopropyl-1H-indole-5-carboxylic acid 10b

In accordance with the synthetic route of Step 4 of Example 9, the starting compound **9c** was replaced with compound **10a**, accordingly, the crude title compound **10b** (21 mg, yield: 100%) was prepared, which was used directly in the next step without purification.
MS m/z (ESI): 396 [M+1].

### Step 3

### (R)-2-(4-Chloro-2-(trifluoromethyl)benzyl)-N-(1-(4-(ethylsulfonyl)phenyl)-2-hydroxyet hyl)-1-isopropyl-1H-indole-5-carboxamide 10

In accordance with the synthetic route of Step 5 of Example 9, the starting compound **9d** was replaced with compound **10b**, accordingly, the title compound **10** (25.2 mg) was prepared.
MS m/z (ESI): 607 [M+1].
¹H NMR (300 MHz, CDCl₃) δ 8.01 (s, 1H), 7.82 (d, 2H), 7.68-7.43 (m, 4H), 7.32 (d, 1H), 7.02 (s, 1H), 6.94 (d, 1H), 6.24 (s, 1H), 5.29 (s, 1H), 4.31 (q, 1H), 4.22 (s, 2H), 4.00 (d, 2H), 3.03 (q, 2H), 1.43 (d, 6H), 1.22 (t, 3H).

### Example 11

### (R)-2-(4-Chloro-2-(trifluoromethyl)benzyl)-1-cyclopropyl-N-(1-(4-(ethylsulfonyl)pheny l)-2-hydroxyethyl)-1H-indole-5-carboxamide 11

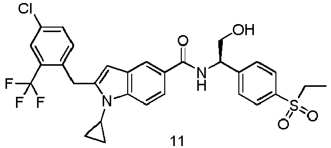

In accordance with the synthetic route of Example 6, the starting compound **5b** was replaced with compound **2b**, accordingly, the title compound **11** (23.8 mg) was prepared.
MS m/z (ESI): 623 [M+1].
¹H NMR (300 MHz, CDCl₃) δ 7.95 (s, 1H), 7.81 (d, 2H), 7.67-7.58 (m, 2H), 7.53 (d, 3H), 7.34 (d, 1H), 7.04 (bs, 1H), 6.95 (d, 1H), 6.13 (s, 1H), 5.25 (m, 1H), 4.34 (s, 2H), 4.12-3.93 (m, 3H), 3.02 (q, 2H), 2.89 (b, 1H), 1.27-1.13 (m, 3H), 1.09-0.88 (m, 4H).

### Example 12

### (R)-2-(4-Chloro-2-(trifluoromethyl)benzyl)-N-(1-(4-(N-cyclopropylsulfamoyl)phenyl)-2 -hydroxyethyl)-1-(2-fluoroethyl)-1H-indole-5-carboxamide 12

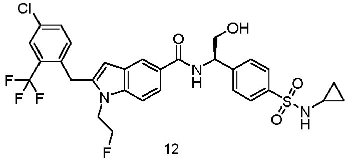

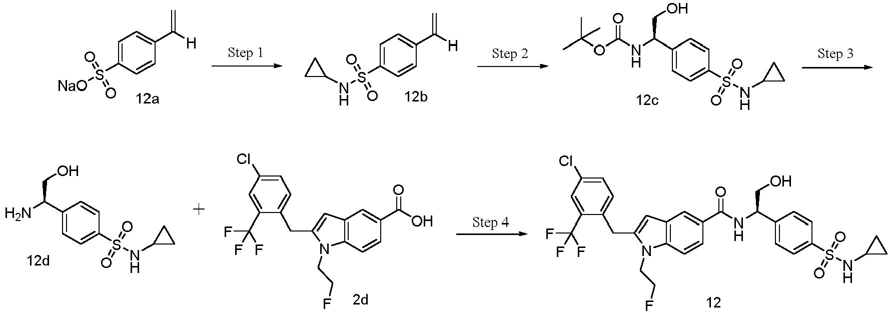

### Step 1

### N-cyclopropyl-4-vinylbenzenesulfonamide 12b

Sodium *p*-styrenesulfonate **12a** was dissolved in 40 mL of toluene. The solution was added with thionyl chloride (11.54 g, 97.00 mmol) and *N*,*N* dimethylformamide (0.5 mL) at room temperature, then heated to 100°C and stirred for 2 hours. The reaction solution was cooled, and concentrated under reduced pressure. The reaction solution was added with dichloromethane (100 mL), and added dropwise with cyclopropylamine (2.22 g, 38.80 mmol). After completion of the addition, the reaction solution was stirred at room temperature for 1 hour, and filtrated. The filtrate was concentrated under reduced pressure, and the resulting residue was purified by silica gel column chromatography with eluent system B to obtain the title compound **12b** (2.0 g, yield: 46.2%).
MS m/z (ESI): 224 [M+1].

### Step 2

### Tert-butyl (R)-(1-(4-(N-cyclopropylsulfamoyl)phenyl)-2-hydroxyethyl)carbamate 12c

Sodium hydroxide (1.07 g, 26.87 mmol) was dissolved in 15 mL of water, and potassium osmate dihydrate (132.00 mg, 358.28 µmol) was dissolved in 5 mL of the resulting solution. *Tert*-butyl carbamate (3.67 g, 31.35 mmol) was dissolved in 100 mL of *n*-propanol at room temperature, and mixed with the above aqueous sodium hydroxide solution. The reaction solution was added dropwise with *tert*-butyl hypochlorite (2.92 g, 26.87 mmol) at room temperature, and stirred for 5 minutes after completion of the addition. The reaction solution was added with hydroquinidine 1,4-phthalazinediyl ether (418.64 mg, 537.42 µmol), and stirred at room temperature for 10 minutes. The reaction solution was added dropwise with 20 mL of the pre-prepared solution of compound **12b** (2.0 g, 8.96 mmol) in *n*-propanol and 5 mL of the sodium hydroxide solution of potassium osmate dihydrate, and stirred at room temperature for 5 hours after completion of the addition. The reaction was quenched with a saturated sodium thiosulfate solution, and the reaction solution was extracted with ethyl acetate (1000 mL×2). The organic phases were combined, dried over anhydrous sodium sulfate, and filtrated. The filtrate was concentrated under reduced pressure, and the resulting residue was purified by silica gel column chromatography with eluent system B to obtain the title compound **12c** (0.3 g, yield: 9.4%).
MS m/z (ESI): 357 [M+1].

### Step 3

### (R)-4-(1-Amino-2-hydroxyethyl)-N-cyclopropylbenzenesulfonamide 12d

Compound **12c** (300 mg, 841.67 µmol) was dissolved in 10 mL of methanol. The solution was added with 4 mL of concentrated hydrochloric acid, and stirred at room temperature for 2 hours. The reaction solution was concentrated under reduced pressure to obtain the title compound **12d** (215 mg, yield: 99.6%).
MS m/z (ESI): 257.4 [M+1].

### Step 4

### (R)-2-(4-Chloro-2-(trifluoromethyl)benzyl)-N-(1-(4-(N-cyclopropylsulfamoyl)phenyl)-2 -hydroxyethyl)-1-(2-fluoroethyl)-1H-indole-5-carboxamide 12

Compound **2d** (100 mg, 250.15 µmol) was dissolved in 2 mL of *N*,*N*-dimethylformamide. The solution was added with compound **12d** (87.89 mg, 300.2 µmol) and *N*,*N*-diisopropylethylamine (64.66 mg, 500.3 µmol), followed by 2-(7-azobenzotriazole)-*N*,*N*,*N*',*N*'-tetramethyluronium hexafluorophosphate (190 mg, 500.3 µmol), and stirred at room temperature for 2 hours. The reaction solution was concentrated under reduced pressure, and the resulting residue was purified by high performance liquid chromatography to obtain the title compound **12** (50 mg, yield: 30.3%).
MS m/z (ESI): 638 [M+1].
¹H NMR (400MHz, CD₃OD) δ 8.69-8.67 (d, 1H), 8.12 (s, 1H), 7.89-7.87 (m, 2H), 7.80 (s, 1H), 7.76-7.73 (d, 1H), 7.68-7.66 (m, 2H), 7.61-7.59 (m, 1H), 7.49-7.47 (m, 1H), 7.28-7.26 (m, 1H), 6.17 (s, 1H), 5.32-5.30 (m, 1H), 4.73 (s, 1H), 4.61 (s, 1H), 4.48 (s, 1H), 4.41-4.40 (m, 3H), 3.95-3.93 (m, 2H), 2.15-2.14 (m, 1H), 1.36-1.31 (m, 1H), 0.55-0.53 (m, 4H).

### Example 13

### (R)-2-(4-Chloro-2-(trifluoromethyl)benzyl)-N-(1-(4-((cyclopropylmethyl)sulfonyl)phen yl)-2-hydroxyethyl)-1-(2-fluoroethyl)-1H-indole-5-carboxamide 13

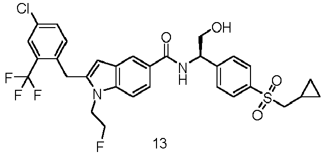

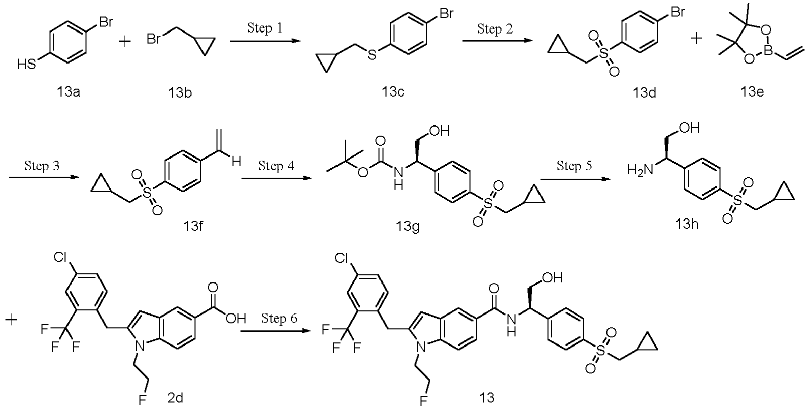

### Step 1

### (4-Bromophenyl)(cyclopropylmethyl)sulfane 13c

4-Bromobenzenethiol **13a** (2.00 g, 10.58 mmol) was dissolved in 15 mL of *N*,*N*-dimethylformamide. The solution was added with potassium carbonate (1.61 g, 11.64 mmol) and bromomethylcyclopropane **13b** (1.57 g, 11.64 mmol), and stirred under an argon atmosphere at room temperature for 16 hours. The reaction solution was filtrated, the filtrate was concentrated under reduced pressure, and the resulting residue was purified by silica gel column chromatography with eluent system B to obtain the title compound **13c** (2.5 g, yield: 97.2%).

### Step 2

### 1-Bromo-4-((cyclopropylmethyl)sulfonyl)benzene 13d

Compound **13c** (2.57 g, 10.58 mmol) was dissolved in 50 mL of dichloromethane. The solution was cooled in an ice bath, then added with *m*-chloroperoxybenzoic acid (4.60 g, 26.42 mmol), and stirred at room temperature for 16 hours. The reaction solution was filtrated, the filtrate was concentrated under reduced pressure, and the resulting residue was purified by silica gel column chromatography with eluent system B to obtain the title compound **13c** (2.9 g, yield: 88.3%).
MS m/z (ESI): 292 [M+18].

### Step 3

### 1-((Cyclopropylmethyl)sulfonyl)-4-vinylbenzene 13f

4-(Cyclopropylmethyl)sulfonylbromobenzene **13d** (2.48 g, 9.01 mmol) was dissolved in 80 mL of 1,4-dioxane. 10 mL of water, vinylboronic acid pinacol ester **13e** (2.78 g, 18.03 mmol) and tetrakis(triphenylphosphine)palladium (520.49 mg, 450.64 µmol) were added, followed by cesium carbonate (5.88 g, 18.03 mmol). The reaction solution was heated to 80°C under an argon atmosphere, and stirred for 2 hours. The reaction solution was concentrated under reduced pressure, and the resulting residue was purified by high performance liquid chromatography to obtain the title compound **13f** (1.83 g, 91.3%).
MS m/z (ESI): 240[M+18].

### Step 4

### Tert-butyl (R)-(1-(4-(cyclopropylmethylsulfonyl)phenyl)-2-hydroxyethyl)carbamate 13g

Sodium hydroxide (1.00 g, 24.70 mmol) was dissolved in 15 mL of water, and potassium osmate dihydrate (121.18 mg, 329.28 µmol) was dissolved in 5 mL of the resulting solution. *Tert*-butyl carbamate (3.38 g, 28.81 mmol) was dissolved in 100 mL of *n*-propanol at room temperature, and mixed with the above aqueous sodium hydroxide solution. The solution was added dropwise with *tert*-butyl hypochlorite (2.68 g, 24.70 mmol) at room temperature, and stirred for 5 minutes after completion of the addition. The reaction solution was added with hydroquinidine 1,4-phthalazinediyl ether (384.64 mg, 493.92 µmol), and stirred at room temperature for 10 minutes. The reaction solution was added dropwise with 20 mL of pre-prepared solution of 4-cyclopropylmethylsulfonylstyrene **13f** (1.83 g, 8.23 mmol) in *n*-propanol and 5 mL of the sodium hydroxide solution of potassium osmate dihydrate, and stirred at room temperature for 5 hours after completion of the addition. The reaction was quenched with a saturated sodium thiosulfate solution, and the reaction solution was extracted with ethyl acetate (1000 mL×2). The organic phases were combined, dried over anhydrous sodium sulfate, and filtrated. The filtrate was concentrated under reduced pressure, and the resulting residue was purified by silica gel column chromatography with eluent system B to obtain the title compound **13g** (1.3 g, yield: 44.43%).
MS m/z (ESI): 256 [M-100+1].

### Step 5

### (R)-2-Amino-2-(4-((cyclopropylmethyl)sulfonyl)phenyl)ethanol 13h

Compound **13g** (1.30 g, 3.66 mmol) was dissolved in 10 mL of methanol. The solution was added with 4 mL of concentrated hydrochloric acid, and stirred at room temperature for 2 hours. The reaction solution was concentrated under reduced pressure to obtain the title compound **13h** (0.9 g, 96.4%).

### Step 6

### (R)-2-(4-Chloro-2-(trifluoromethyl)benzyl)-N-(1-(4-((cyclopropylmethyl)sulfonyl)phen yl)-2-hydroxyethyl)-1-(2-fluoroethyl)-1H-indole-5-carboxamide 13

Compound **2d** (200 mg, 500.29 µmol) was dissolved in 5 mL of *N*,*N*-dimethylformamide. Compound **13h** (218.97 mg, 750.44 µmol) and *N*,*N*-diisopropylethylamine (129.32 mg, 1.00 mmol), followed by 2-(7-azobenzotriazole)-*N*,*N*,*N*',*N*'-tetramethyluronium hexafluorophosphate (235.41 mg, 1.00 mmol) were added. The reaction solution was stirred at room temperature for 2 hours. The reaction solution was concentrated under reduced pressure, and the resulting residue was purified by high performance liquid chromatography to obtain the title compound **13** (41 mg, 12.8%).
MS m/z (ESI): 637 [M+1].
¹H NMR (400 MHz, CDCl₃) δ 8.13-8.12 (d, 1H), 7.97-7.95 (m, 2H), 7.76-7.75 (m, 1H), 7.65-7.63 (m, 2H), 7.47-7.45 (m, 1H), 7.39-7.38 (d, 1H), 7.20-7.16 (m, 1H), 7.13-7.11 (m, 1H), 6.30 (s, 1H), 5.39-5.35 (m, 1H), 4.70-4.68 (t, 1H), 4.59-4.56 (t, 1H), 4.38-4.35 (m, 3H), 4.31-4.29 (m, 1H), 4.15-4.11 (dd, 1H), 4.08-4.04 (dd, 1H), 3.05-3.04 (d, 2H), 1.09-1.05 (m, 1H), 0.64-0.59 (m, 2H), 0.24-0.20 (m, 2H).

### Example 14

### (R)-N-(1-(4-((Cyclopropylmethyl)sulfonyl)phenyl)-2-hydroxyethyl)-2-(4-fluoro-2-(trifl uoromethyl)benzyl)-1-(2-fluoroethyl)-1H-indole-5-carboxamide 14

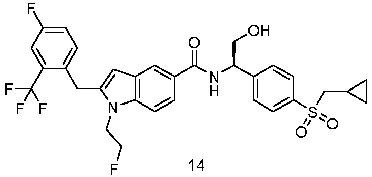

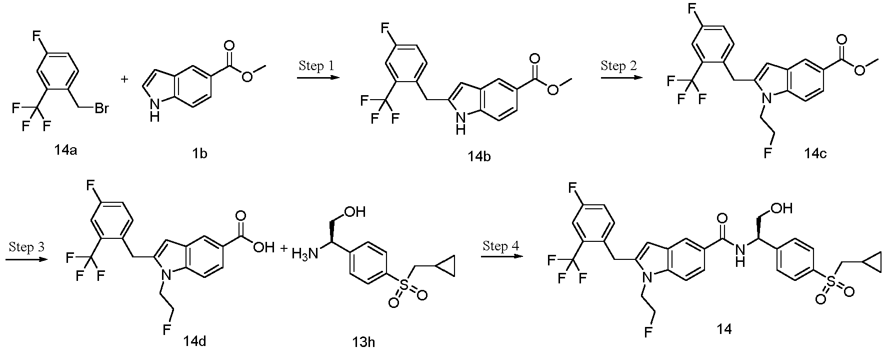

### Step 1

### Methyl 2-(4-fluoro-2-(trifluoromethyl)benzyl)-1H-indole-5-carboxylate 14b

4-Fluoro-2-trifluoromethylbenzyl bromide **14a** (2.29 g, 8.90 mmol), compound **1b** (1.30 g, 7.42 mmol), bis(acetonitrile)palladium(II) chloride (385.05 mg, 1.48 mmol), bicyclo[2.2.1]-2-heptene (698.67 mg, 7.42 mmol) and potassium carbonate (1.57 g, 14.84 mmol) were added to 20 mL of *N*,*N-*dimethylacetamide. The reaction solution was heated to 80°C and stirred for 16 hours under an argon atmosphere. The reaction solution was cooled to room temperature and filtrated. The filtrate was poured into water, and extracted with ethyl acetate. The organic phases were combined, dried over anhydrous sodium sulfate, and filtrated. The filtrate was concentrated under reduced pressure, and the resulting residue was purified by silica gel column chromatography with eluent system B to obtain the title compound **14b** (2.0 g, yield: 76.7%).
MS m/z (ESI): 350 [M-1].

### Step 2

### Methyl 2-(4-fluoro-2-(trifluoromethyl)benzyl)-1-(2-fluoroethyl)-1H-indole-5-carboxylate 14c

Compound **14b** (140 mg, 398.53 µmol), 1-fluoro-2-iodo-ethane (151.2 mg, 1.20 mmol) and cesium carbonate (389.54 mg, 1.20 mmol) were added to 15 mL of acetonitrile. The reaction solution was reacted under a microwave condition at 100°C for one hour. The reaction solution was poured into water, and extracted with ethyl acetate. The organic phases were combined, dried over anhydrous sodium sulfate, and filtrated. The filtrate was concentrated under reduced pressure, and the resulting residue was purified by silica gel column chromatography with eluent system B to obtain the title compound **14c** (135 mg, yield: 85.25%).
MS m/z (ESI): 396 [M-1].

### Step 3

### 2-(4-Fluoro-2-(trifluoromethyl)benzyl)-1-(2-fluoroethyl)-1H-indole-5-carboxylic acid 14d

Compound **14c** (135 mg, 339.76 µmol) and sodium hydroxide (37.05 mg, 926.39 µmol) were added to a mixed solvent of 6 mL of methanol and 0.5 mL of water. The reaction solution was stirred at 60°C for 2 hours. Methanol was removed under reduced pressure, and the resulting residue was added dropwise with 1*M* diluted hydrochloric acid to adjust the pH to ∼3. The reaction solution was extracted with ethyl acetate, and the organic phase was dried over anhydrous sodium sulfate, and filtrated. The filtrate was concentrated under reduced pressure to obtain the crude title compound **14d** (130 mg), which was used directly in the next step without purification.
MS m/z (ESI): 382 [M-1].

### Step 4

### (R)-N-(1-(4-((Cyclopropylmethyl)sulfonyl)phenyl)-2-hydroxyethyl)-2-(4-fluoro-2-(trifl uoromethyl)benzyl)-1-(2-fluoroethyl)-1H-indole-5-carboxamide 14

Compound **14d** (300 mg, 782.65 µmol) was dissolved in 5 mL of *N*,*N*-dimethylformamide. Compound **13h** (342.56 mg, 1.17 mmol) and *N*,*N*-diisopropylethylamine (202.3 mg, 1.57 mmol) were added, followed by 2-(7-azobenzotriazole)-*N*,*N*,*N*',*N*'-tetramethyluronium hexafluorophosphate (594.82 mg, 1.57 mmol). The reaction solution was stirred at room temperature for 2 hours. The reaction solution was concentrated under reduced pressure, and the resulting residue was purified by high performance liquid chromatography to obtain the title compound **14** (200 mg, 41.2%).
MS m/z (ESI): 621 [M+1].
¹H NMR (400 MHz, CDCl₃) δ 8.13-8.12 (d, 1H), 7.94-7.92 (m, 2H), 7.77-7.74 (m, 1H), 7.63-7.61 (m, 2H), 7.50-7.47 (m, 1H), 7.36-7.34 (d, 1H), 7.25-7.23 (m, 1H), 7.20-7.14 (m, 1H), 6.30 (s, 1H), 5.39-5.35 (m, 1H), 4.69-4.67 (t, 1H), 4.58-4.55 (t, 1H), 4.38-4.35 (m, 3H), 4.32-4.29 (m, 1H), 4.12-4.08 (dd, 1H), 4.05-4.01 (dd, 1H), 3.05-3.02 (d, 2H), 1.05-0.98 (m, 1H), 0.64-0.59 (m, 2H), 0.24-0.20 (m, 2H).

### Example 15

### (R)-2-(4-Chloro-2-(trifluoromethyl)benzyl)-N-(1-(4-(cyclopropylsulfonyl)phenyl)-2-hy droxyethyl)-1-(2-fluoroethyl)-1H-indole-5-carboxamide 15

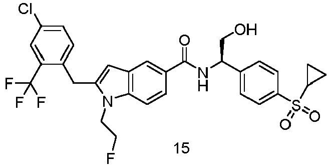

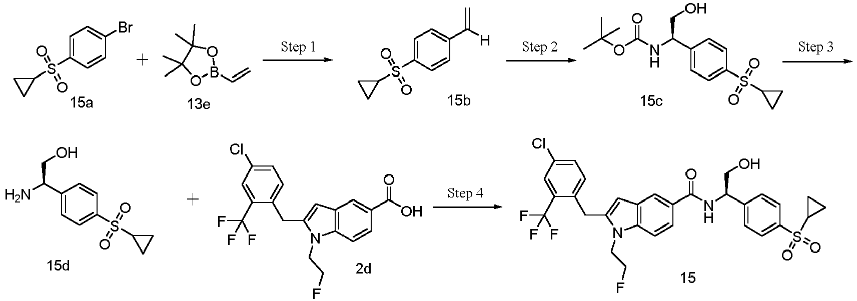

### Step 1

### 1-((Cyclopropyl)sulfonyl)-4-vinylbenzene 15b

4-Cyclopropylsulfonylbromobenzene **15a** (315 mg, 1.21 mmol) was dissolved in 20 mL of 1,4-dioxane. 5 mL of water, compound **13e** (223 mg, 1.45 mmol) and tetrakis(triphenylphosphine)palladium (55.8 mg, 48.25 µmol) were added, followed by cesium carbonate (786.5 mg, 2.41 mmol). The reaction solution was heated to 80°C under an argon atmosphere, and stirred for 2 hours. The reaction solution was concentrated under reduced pressure, and the resulting residue was purified by high performance liquid chromatography to obtain the title compound **15b** (210 mg, 83.6%).
MS m/z (ESI): 226 [M+18].

### Step 2

### Tert-butyl (R)-(1-(4-(cyclopropylsulfonyl)phenyl)-2-hydroxyethyl)carbamate 15c

Sodium hydroxide (121 mg, 3.02 mmol) was dissolved in 15 mL of water, and potassium osmate dihydrate (14.84 mg, 40.33 µmol) was dissolved in 5 mL of the resulting solution. *Tert*-butyl carbamate (413.3 mg, 3.53 mmol) was dissolved in 10 mL of *n*-propanol at room temperature, and mixed with the above aqueous sodium hydroxide solution. The reaction solution was added dropwise with *tert*-butyl hypochlorite (328.4 mg, 3.02 mmol) at room temperature, and stirred for 5 minutes after completion of the addition. The reaction solution was added with hydroquinidine 1,4-phthalazinediyl ether (47.13 mg, 60.5 µmol), and stirred at room temperature for 10 minutes. The reaction solution was added dropwise with 10 mL of a solution of compound **15b** (0.21 g, 1.01 mmol) in *n*-propanol and 5 mL of the sodium hydroxide solution of potassium osmate dihydrate, and stirred at room temperature for 5 hours after completion of the addition. The reaction was quenched with a saturated sodium thiosulfate solution, and the reaction solution was extracted with ethyl acetate (50 mL×2). The organic phases were combined, dried over anhydrous sodium sulfate, and filtrated. The filtrate was concentrated under reduced pressure, and the resulting residue was purified by silica gel column chromatography with eluent system B to obtain the title compound **15c** (133 mg, yield: 38.6%).
MS m/z (ESI): 242 [M-100+1].

### Step 3

### (R)-2-Amino-2-(4-((cyclopropyl)sulfonyl)phenyl)ethanol 15d

Compound **15c** (133 mg, 389.56 µmol) was dissolved in 10 mL of methanol. The solution was added with 4 mL of concentrated hydrochloric acid, and stirred at room temperature for 2 hours. The reaction solution was concentrated under reduced pressure to obtain the title compound **15d** (90 mg, 96.4%).

### Step 4

### (R)-2-(4-Chloro-2-(trifluoromethyl)benzyl)-N-(1-(4-(cyclopropylsulfonyl)phenyl)-2-hy droxyethyl)-1-(2-fluoroethyl)-1H-indole-5-carboxamide 15

Compound **2d** (150 mg, 375.22 µmol) was dissolved in 5 mL of *N*,*N*-dimethylformamide. Compound **15d** (90.54 mg, 375.22 µmol) and *N*,*N*-diisopropylethylamine (97.00 mg, 750.44 µmol) were added, followed by 2-(7-azobenzotriazole)-*N*,*N*,*N*',*N*'-tetramethyluronium hexafluorophosphate (285.41 mg, 750.44 µmol). The reaction solution was stirred at room temperature for 2 hours. The reaction solution was concentrated under reduced pressure, and the resulting residue was purified by high performance liquid chromatography to obtain the title compound **15** (100 mg, 42.8%).
MS m/z (ESI): 621 [M-1].
¹H NMR (400 MHz, CDCl₃) δ 8.13 (s, 1H), 7.94-7.92 (m, 2H), 7.77-7.75 (m, 2H), 7.64-7.62 (m, 2H), 7.48-7.45 (m, 1H), 7.38-7.36 (m, 1H), 7.15-7.12 (m, 2H), 6.34 (s, 1H), 5.41-5.38 (m, 1H), 4.70-4.68 (t, 1H), 4.60-4.56 (t, 1H), 4.38-4.36 (m, 3H), 4.31-4.29 (m, 1H), 4.16-4.12 (dd, 1H), 4.09-4.05 (dd, 1H), 2.51-2.46 (m, 1H), 1.41-1.37 (m, 2H), 1.09-1.05 (m, 2H).

### Example 16

### (R)-2-(4-Chloro-2-(trifluoromethyl)benzyl)-N-(1-(4-(ethylsulfonyl)phenyl)-2-methoxyet hyl)-1-(2-fluoroethyl)-1H-indole-5-carboxamide 16

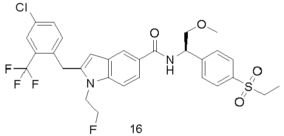

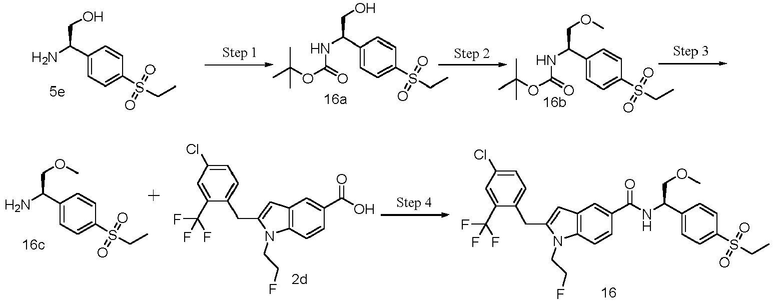

### Step 1

### Tert-butyl (R)-(1-(4-(ethylsulfonyl)phenyl)-2-hydroxyethyl)carbamate 16a

Compound **5e** (50 mg, 218.06 µmol) was dissolved in 6 mL of dichloromethane. The solution was added with triethylamine (44.63 mg, 436.12 µmol) and di-*tert*-butyl dicarbonate (95.07 mg, 436.12 µmol) at 0°C, and stirred for one hour. The reaction was quenched by adding ice water. The reaction solution was extracted with ethyl acetate, and the organic phase was dried over anhydrous sodium sulfate, and filtrated. The filtrate was concentrated under reduced pressure, and the crude product was purified by thin layer chromatography with eluent system B to obtain compound **16b** (45 mg, yield: 62.6%).
MS m/z (ESI): 230.2 [M-100+1].

### Step 2

### Tert-butyl (R)-(1-(4-(ethylsulfonyl)phenyl)-2-methoxyethyl)carbamate 16b

Compound **16a** (50 mg, 151.79 µmol) was dissolved in 6 mL of tetrahydrofuran. The solution was added with sodium hydride (11.63 mg, 303.57 µmol) at 0°C, and stirred for 10 minutes. The reaction solution was added with methyl iodide (23.70 mg, 166.96 µmol), and stirred for 2 hours. The reaction was quenched by adding ice water. The reaction solution was extracted with ethyl acetate, and the organic phase was dried over anhydrous sodium sulfate, and filtrated. The filtrate was concentrated under reduced pressure, and the crude product was purified by thin layer chromatography with eluent system B to obtain compound **16b** (45 mg, yield: 86.32%).

### Step 3

### (R)-1 -(4-(ethylsulfonyl)phenyl)-2-methoxyethylamine 16c

Compound **16b** (45 mg, 131.03 µmol) was dissolved in 10 mL of dichloromethane. The solution was added with trifluoroacetic acid (298.80 mg, 2.62 mmol), and stirred at room temperature for 16 hours. The reaction solution was concentrated under reduced pressure to obtain the crude title compound **16c** (40 mg), which was used directly in the next step without purification.

### Step 4

### (R)-2-(4-Chloro-2-(trifluoromethyl)benzyl)-N-(1-(4-(ethylsulfonyl)phenyl)-2-methoxyet hyl)-1-(2-fluoroethyl)-1H-indole-5-carboxamide 16

Compound **2d** (44.75 mg, 111.94 µmol) and the crude compound **16c** (40.00 mg, 111.94 µmol) were dissolved in 20 mL of *N*,*N*-dimethylformamide. The solution was added with 2-(7-azobenzotriazole)-*N*,*N*,*N*',*N*'-tetramethyluronium hexafluorophosphate (31.60 mg, 134.32 µmol) and *N*,*N*-diisopropylethylamine (43.40 mg, 335.81 µmol), and stirred at room temperature for 16 hours. The reaction solution was added with water, and extracted with ethyl acetate. The organic phases were combined, dried over anhydrous sodium sulfate, and filtrated. The filtrate was concentrated under reduced pressure, and the resulting residue was purified by silica gel column chromatography with eluent system B to obtain the title compound **16** (25 mg, yield: 35.73%).
MS m/z (ESI): 625.6 [M+1].
¹H NMR (400MHz, CDCl₃) δ 8.07 (s, 1H), 7.87-7.85 (d, 2H), 7.73-7.71 (m, 2H), 7.62-7.60 (m, 2H), 7.43-7.41 (m, 1H), 7.34-7.31 (m, 1H), 7.15-7.08 (m, 2H), 6.28 (s, 1H), 5.44-5.40 (m, 1H), 4.66-4.64 (m, 1H), 4.55-4.52 (m, 1H), 4.34-4.29 (m, 3H), 4.28-4.25 (m, 1H), 3.83-3.74 (m, 2H), 3.39 (s, 3H), 3.12-3.06 (m, 2H), 1.29-1.26 (m, 3H).

### Example 17 (Comparative Example)

### (R)-2-(4-(trifluoromethyl)benzyl)-N-(1-(4-(ethylsulfonyl)phenyl)-2-hydroxyethyl)-1-(2-fluoroethyl)-1H-indole-5-carboxamide 17

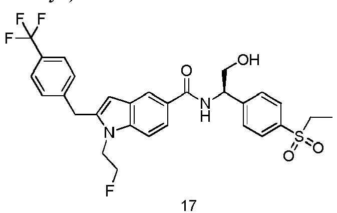

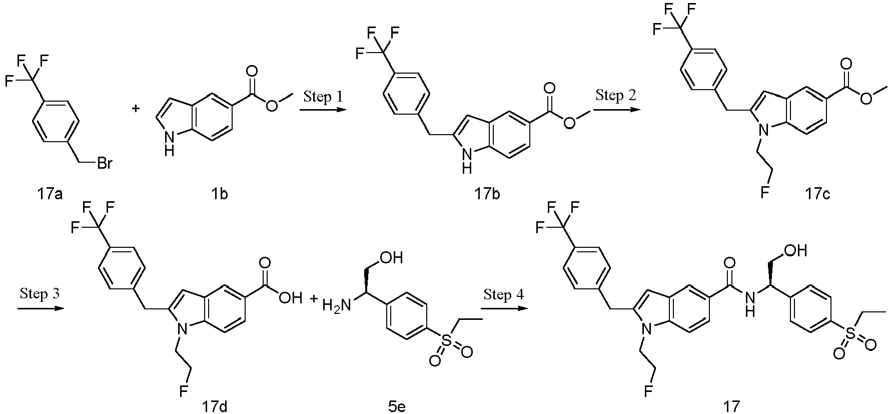

### Step 1

### Methyl 2-(4-(trifluoromethyl)benzyl)-1H-indole-5-carboxylate 17b

Compound **1b** (1.00 g, 5.71 mmol) and 4-(trifluoromethyl)benzyl bromide **17a** (16.40 g, 6.86 mmol) were dissolved in 15 mL of *N*,*N*-dimethylacetamide. Bis(acetonitrile)palladium(II) chloride (296.17 mg, 1.14 mmol), bicyclo[2.2.1]-2-heptene (1.1 g, 11.68 mmol) and sodium carbonate (1.22 g, 11.51 mmol) were added. The reaction solution was heated to 80°C and stirred for 17 hours under an argon atmosphere. The reaction solution was cooled and filtrated. The filtrate was concentrated under reduced pressure, and the resulting residue was purified by silica gel column chromatography with eluent system B to obtain the title compound **17b** (1.60 g, yield: 84.10%).
MS m/z (ESI): 334.1 [M+1].

### Step 2

### Methyl 2-(4-(trifluoromethyl)benzyl)-1-(2-fluoroethyl)-1H-indole-5-carboxylate 17c

Compound **17b** (500 mg, 1.50 mmol), 1-fluoro-2-iodo-ethane (381.0 mg, 3.0 mmol) and cesium carbonate (976 mg, 2.0 mmol) were added to 10 mL of acetonitrile. The reaction solution was reacted under a microwave condition at 100°C for one hour. The reaction solution was poured into water, and extracted with ethyl acetate. The organic phases were combined, dried over anhydrous sodium sulfate, and filtrated. The filtrate was concentrated under reduced pressure, and the resulting residue was purified by silica gel column chromatography with eluent system B to obtain the title compound **17c** (500 mg, yield: 87.86%).
MS m/z (ESI): 380.1 [M+1].

### Step 3

### 2-(4-(Trifluoromethyl)benzyl)-1-(2-fluoroethyl)-1H-indole-5-carboxylic acid 17d

Compound **17c** (500 mg, 1.32 mmol) and sodium hydroxide (527.2 mg, 13.18 mmol) were added to a mixed solvent of 10 mL of methanol and 2 mL of water. The solution was stirred at 60°C for 2 hours. Methanol was removed under reduced pressure, and the resulting residue was added dropwise with 1*M* diluted hydrochloric acid to adjust the pH to ∼3. The solution was extracted with ethyl acetate, and the organic phase was dried over anhydrous sodium sulfate, and filtrated. The filtrate was concentrated under reduced pressure to obtain the crude title compound **17d** (500 mg), which was used directly in the next step without purification.
MS m/z (ESI): 366.1 [M+1].

### Step 4

### (R)-2-(4-(trifluoromethyl)benzyl)-N-(1-(4-(ethylsulfonyl)phenyl)-2-hydroxyethyl)-1-(2-fluoroethyl)-1H-indole-5-carboxamide 17

(*R*)-2-Amino-2-(4-(ethylsulfonyl)phenyl)ethanol **5e** (76 mg, 0.33mmol), the crude compound **17d** (100 mg, 0.27 mmol), O-(7-azabenzotriazol-1-yl)-*N*,*N*,*N*',*N*'-tetramethyluronium hexafluorophosphate (125 mg, 0.33 mmol) and *N*,*N*-diisopropylethylamine (54 mg, 0.42 mmol) were added to 5 mL of *N*,*N*-dimethylformamide. The reaction solution was stirred at room temperature for 16 hours. The reaction solution was extracted with ethyl acetate, and the organic phases were combined, dried over anhydrous sodium sulfate, and filtrated. The filtrate was concentrated under reduced pressure, and the resulting residue was purified by silica gel column chromatography with eluent system B to obtain the title compound **17** (46 mg, yield: 29.14%).
MS m/z (ESI): 577.1 [M+1].
¹H NMR (400MHz, CD₃OD) δ 8.65 (d, 1H), 8.12 (s, 1H), 7.88 (d, 2H), 7.86 (d, 3H), 7.63 (d, 2H), 7.46 -7.43 (m 3H), 6.3 (s, 1H), 5.3 (d, 1H), 4.64-4.62 (t, 1H), 4.52-4.5 (t, 1H), 4.47-4.45 (t, 1H), 4.41-4.38 (t, 1H), 4.10(d, 2H), 3.92(d, 2H), 3.22-3.16 (m 2H), 1.26-1.19 (m, 3H).

### Biological Assay

The present invention will be further described with reference to the following test examples, but the examples should not be considered as limiting the scope of the present invention.

### Test Example 1. Determination of the effect of the compounds of the present invention on the in vitro activity of RORγ

### I. Experimental materials and instruments

1. LanthaScreen® TR-FRET RORγ co-activation system (Life Technologies)
2. RORγ LBD (AB Vector)
3. DMSO (SigmaAldrich)
4. Microplate reader (Tecan)

### II. Experimental procedures

The regulation of the compounds of the present invention on RORγ activity was screened with a LanthaScreen TR-FRET (Time Resolved Fluorescence Resonance Energy Transfer) RORγ co-activation system.

A complete buffer D (complete TR-FRET Coregulator) (Life Technologies) was formulated first, containing a final concentration of 5 mM DTT. The final concentration of DMSO was 2%. The test compound was serially diluted to 2x final concentration in the complete buffer D containing 2% of DMSO, and the maximum dose was 60 µm. The test compound was added to the test wells of a 384-well plate (PerkinElmer) in 10 µl/well. Two parallel control wells were set up for each test compound at the same concentration. 4X RORγ LBD (AB Vector) was formulated. RORγ LBD was diluted with the complete buffer D to a concentration of 1 ng/µL, and added to the test wells of 384-well plate in 5 µl/well. The negative control well was 5 µL of complete buffer D without RORγ LBD. A mixed solution comprising 0.6 µM of fluorescein-D22 (4X) and 8 nM of terbium(Tb)-labeled anti-GST antibody (4X) (Life Technologies) was formulated with the complete buffer D, and 5 µL of the mixed solution was added to the 384-well plate. The total reaction system was 20 µL. The 384-well plate was gently shaken on a shaker, and incubated at room temperature in the dark for 2-4 hours.

Fluorescence readings were determined with Tecan Infinite M1000. The logarithmic curve of the ratio of the emission wavelength of 520 nm/495 nm to the concentration of the compound was plotted by GraphPad Prism 6.0 software. EC₅₀/IC₅₀ value of the test compound was calculated.

The effect of the compounds of the present invention on the *in vitro* activity of RORγ was determined by the above test, and the resulting EC₅₀ values are shown in Table 1.

**Table 1 EC₅₀ values of the compounds of the present invention and IC₅₀ value of the Comparative Example on the in vitro activity of RORγ.**

| Example No. | EC₅₀/IC₅₀ ^{a} (nM) | Emax(%)/maximum inhibition rate^{b} | Type |
|---|---|---|---|
| 1 | 64 | 94% | Agonist |
| 2 | 116 | 68% | Agonist |
| 4 | 15 | 107% | Agonist |
| 5 | 334 | 110% | Agonist |
| 6 | 79 | 105% | Agonist |
| 7 | 80 | 84% | Agonist |
| 11 | 22 | 94% | Agonist |
| 12 | 13 | 89% | Agonist |
| 13 | 69 | 96% | Agonist |
| 14 | 138 | 99% | Agonist |
| 15 | 124 | 109% | Agonist |
| 16 | 18 | 97% | Agonist |
| 17 | 29 | 67% | Inverse agonist |

| | | | |
|---|---|---|---|
| a: For agonist, the value refers to EC₅₀; for inverse agonist, the value refers to IC₅₀; b: For agonist, the value refers to Emax(%); for inverse agonist, the value refers to maximum inhibition rate. | | | |

Conclusion: The compounds of the present invention have a significant agonistic effect on the *in vitro* activity of RORγ. Meanwhile, the applicant found that changes of the ortho group of ring A can alter its regulation effect, and the compound of Example 17, in which the ortho group of ring A is a group having a small steric hindrance (such as H), is an inverse agonist.

### Test Example 2. Determination of the activity of the compounds of the present invention on IL-17A by enzyme-linked immune quantitative assay

### I. Experimental materials and instruments

1. Human peripheral blood mononuclear cells (PBMC) (Zenbio)
2. Lymphocyte culture medium (Zenbio)
3. TexMACS (Miltenyi Biotec)
4. Human cytostim (Miltenyi Biotec)
5. Human IL-17 enzyme-linked immunosorbent kit (R&D System)
6. CO₂ incubator (Fisher Scientific)
7. Centrifuge (Fisher Scientific)
8. 96-well cell culture plate (Fisher Scientific)
9. Microplate reader (Tecan)

### II. Experimental procedures

Frozen human peripheral blood mononuclear cells (PBMC) were rapidly resuscitated in pre-warmed lymphocyte culture medium, and centrifuged at 1000 rpm for 10 min. The cell culture supernatant was removed. The cells were gently suspended in TexMACS medium and counted. The T cell activation reagent cytostim (10 µl/ml) was added in proportion to the cell suspension. Then, the cells were seeded in a 96-well cell culture plate at a density of 1 × 10⁵ peripheral blood mononuclear cells/well. The test compounds were diluted in gradient with TexMACS medium, and added respectively to the test wells, with 2-3 parallel wells per group. A negative control well containing only cells without cytostim was provided to obtain the background reading. The cell culture plate was placed in a incubator at 5% carbon dioxide, 37°C to incubate for 3 days. The cell culture supernatant was collected 3 days after drug treatment, and centrifuged to remove the suspension. Then, IL-17A in the supernatant was quantified with IL-17A enzyme-linked immunosorbent kit. EC50 values of the test compounds were calculated with GraphPad Prism 6.0.

The effect of the compounds of the present invention on IL-17A by enzyme-linked immune quantitative assay was determined by the above test, and the resulting EC₅₀ values are shown in Table 2.

**Table 2 EC₅₀ values of the compounds of the present invention on IL-17A by enzyme-linked immune quantitative assay**

| Example No. | EC₅₀ (nM) | Emax(%) |
|---|---|---|
| 2 | 90 | 82% |
| 3 | 169 | 136% |
| 4 | 85 | 93% |
| 11 | 276 | 72% |
| 12 | 25 | 71% |
| 13 | 27 | 65% |
| 14 | 42 | 99% |
| 16 | 8 | 99% |

Conclusion: The compounds of the present invention have a significant regulation effect on IL-17A by enzyme-linked immune quantitative assay.

### Pharmacokinetics Evaluation

### Test Example 3. Pharmacokinetics assay of the compound of the present invention in mice

### 1. Abstract

Mice were used as test animals. The drug concentration in plasma at different time points was determined by LC/MS/MS method after intragastrical administration of the compound of Example 4 to mice. The pharmacokinetic behavior of the compound of the present invention was studied and evaluated in mice.

### 2. Test protocol

### 2.1 Test compound

Compound of Example 4.

### 2.2 Test animals

A group of nine C57 mice (female) were purchased from Shanghai Jiesijie Laboratory Animal Co., LTD, with Certificate No.: SCXK (Shanghai) 2013-0006.

### 2.3 Preparation of the test compound

A certain amount of the test compound was weighed, and added with 5% by volume of DMSO, 5% by volume of tween 80 and 90% by volume of normal saline to prepare a 0.1 mg/mL colorless, clear and transparent solution.

### 2.4 Administration

After an overnight fast, C57 mice were administrated intragastrically with the test compound at an administration dose of 2.0 mg/kg and an administration volume of 0.2 mL/10 g.

### 3. Process

The mice were intragastrically administered the compound of Example 4. 0.1 ml of blood was taken (from 3 animals at each time point) before administration and at 0.25, 0.5, 1.0, 2.0, 4.0, 6.0, 8.0, 11.0 and 24.0 hours after administration. The samples were stored in heparinized tubes, and centrifuged for 10 minutes at 3500 rpm to separate the blood plasma. The plasma samples were stored at -20°C.

The content of the test compound in the plasma of mice after intragastrical administration of the test compound at different concentrations was determined: 25 µL of mouse plasma at each time after administration was taken, added with 80 µL of the internal standard solution of camptothecin (100 ng/mL) and 200 µL of acetonitrile, vortex-mixed for 5 minutes, and centrifuged for 10 minutes (3600 rpm). 1 µL of the supernatant was taken from the plasma samples for LC/MS/MS analysis.

### 4. Results of pharmacokinetic parameters

Pharmacokinetic parameters of the compound of the present invention are shown below:

| No. | Pharmacokinetics assay in mice (2mg/kg) | | | | | |
|---|---|---|---|---|---|---|
| | Plasma concentration | Area under curve | Half-life | Residence time | Clearance | Apparent distribution volume |
| | Cmax (ng /mL) | AUC (ng /mL^{∗}h) | T1/2 (h) | MRT (h) | CLz/F (ml/min/kg) | Vz/F (ml/kg) |
| Example 4 | 3660 | 50554 | 11.2 | 15.5 | 0.66 | 637 |

Conclusion: The compound of the present invention is well absorbed, and has a pharmacokinetic advantage.

### Pharmacodynamic Evaluation

### Test Example 4. Efficacy of RORy agonist in isotype MC38 colorectal tumor mice model

### 1. Experimental purpose

The inhibition effect of the compound of Example 4 on MC38 tumor growth was evaluated in MC38 mice model.

### 2. Experimental method and experimental materials

### 2.1. Test animals and feeding conditions

Experimental female C57BL/6 mice were purchased from Charles River Lab (U.S.A.). The mice weighed 20-25 gram, and were 7-9 weeks old when purchased. The mice (10 mice per cage) were maintained in a constant temperature of 23±1°C, and a humidity of 50-60%, and free access to food and water. The mice were treated and used in accordance with the Institutional Animal Care and Use Committee (IACUC approved guidelines). After the animals were purchased, the test was started after 7 days of adaptive feeding.

### 2.2. Experimental drugs

Compound of Example 4;
Anti-mouse PD-1 (CD279) antibody, purchased from BioXcell (clone RMP1-14; catalog number BP0146);
IgG2a isotype control antibody, purchased from BioXcell (clone 2A3; catalog number BE0089).

### 2.3. Experimental design and experimental method

### 2.3.1. Animal grouping:

After adaptive feeding, the mice were grouped as follows:

| Groups | n | Administration mode | Administration regimen |
|---|---|---|---|
| IgG2a isotype control antibody plus vehicle control group | 8 | Intraperitoneal injection/oral administration | Q3dx4/BIDx21 |
| Anti-mouse PD-1 antibody | 8 | Intraperitoneal injection | Q3dx4 |
| Compound of Example 4 | 8 | Oral administration | BIDx21 |
| Anti-mouse PD-1 antibody plus compound of Example 4 | 8 | Intraperitoneal injection/oral administration | Q3dx4/BIDx21 |

| | | | |
|---|---|---|---|
| Note: 1. Q3dx4 refers to administration every three days for a total of four times, and the administration is fixed on Day 5, 8, 11 and 14; | | | |

### 2. BIDx21 refers to administration twice a day for 21 consecutive days.

### 2.3.2. Experimental method

Female C57BL/6 mice (20-25 gram, 7-9 weeks old) were used in the experiment. *In vivo* antitumor activity of the compound of Example 4 administrated alone or the compound of Example 4 administrated in combination with anti-mouse PD-1 antibody was evaluated by detecting the growth of isotype MC38 colorectal tumor (Synta Pharmaceuticals) in inbred C57BL/6 mice. 500,000 (5×10⁵) MC38 cells were implanted subcutaneously in the right abdomen of each mouse. After 5 days, when the tumor grew to 40-80 mm³, the mice were grouped randomly. The compound of Example 4 (30 mg/kg) was administrated twice a day for 21 consecutive days. During the treatment experiment in which the antibody administrated alone or in combination with the compound of Example 4, anti-mouse PD-1 (CD279) antibody (BioXcell) (5 mg/kg) was intraperitoneally injected (i.p.) to the mice bearing MC38 tumor fixedly on Day 5, 8, 11 and 14. The control group was administrated with the vehicle CMC-Na drug formulation and the IgG2a isotype control antibody.

### 2.4. Data presentation:

The tumor volume was measured with a caliper in three dimensions, and then calculated according to the following formula: tumor volume (mm³) = 1 × w × h × 0.5236, wherein 1 represents the length of the tumor, w represents the width of the tumor, and h represents the height of the tumor, in millimeters. Tumor growth inhibition rate TGI% = 100 × (TV_{control} - TVₜᵤₘₒᵣ) / (TV_{control} - TVᵢₙᵢₜᵢₐₗ), wherein TV_{control} = the tumor volume of the control group; TVₜᵤₘₒᵣ = the tumor volume of the treatment group; and TVᵢₙᵢₜᵢₐₗ = the initial tumor volume on Day 5.

### 3. Results and discussion:

As shown in Figure 1, when 30 mg/kg of the compound of Example 4 was administrated alone, the TGI was 40%. When the anti-mouse PD-1 (CD279) antibody (5 mg/kg) was injected alone, the TGI was 51%. When being administrated in combination with the anti-mouse PD-1 monoclonal antibody (5 mg/kg), the compound of Example 4 (30 mg/kg) exhibited a synergistic effect (the TGI was 63%). These data indicate that in the isogenic MC38 colorectal tumor model, the administration of the compound of Example 4 alone exhibits an antitumor activity, and the combined administration of the compound of Example 4 and PD-1 antibody exhibits a synergistic effect. These data also indicate that the compound of Example 4 has a biological activity consistent with RORγ activation (rather than inhibition), openning up a novel way of improving the efficacy of immunotherapy.

## Claims

1. A compound of formula (I): 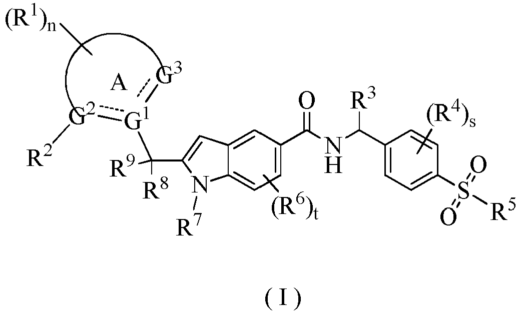 or a tautomer, mesomer, racemate, enantiomer, diastereomer thereof, or mixture thereof, or a pharmaceutically acceptable salt thereof,
wherein:
= is a double bond or single bond;
G¹, G² and G³ are identical or different and are each independently selected from the group consisting of C, CH, CH₂ and N;
ring A is selected from the group consisting of aryl, heteroaryl, cycloalkyl and heterocyclyl;
each R¹ is identical or different and each is independently selected from the group consisting of hydrogen, halogen, alkyl, haloalkyl, alkoxy, haloalkoxy, cyano, amino, nitro, hydroxy and hydroxyalkyl;
R² is a haloalkyl;
R³ is selected from the group consisting of alkyl, haloalkyl, alkoxy, haloalkoxy, hydroxyalkyl, halogen, cyano, amino, nitro, hydroxy, cycloalkyl, heterocyclyl, aryl and heteroaryl, wherein the alkyl, haloalkyl, cycloalkyl, heterocyclyl, aryl and heteroaryl are each independently optionally substituted by one or more substituents selected from the group consisting of hydroxy, halogen, alkyl, alkoxy and amino;
each R⁴ is identical or different and each is independently selected from the group consisting of hydrogen, halogen, alkyl, haloalkyl, alkoxy, haloalkoxy, cyano, amino, nitro, hydroxy, hydroxyalkyl, cycloalkyl, heterocyclyl, aryl and heteroaryl;
R⁵ is selected from the group consisting of hydrogen, alkyl, haloalkyl, amino, hydroxy, hydroxyalkyl, cycloalkyl, heterocyclyl, NR¹⁰R¹¹, aryl and heteroaryl, wherein the alkyl, cycloalkyl, heterocyclyl, aryl and heteroaryl are each independently optionally substituted by one or more substituents selected from the group consisting of hydroxy, halogen, alkyl, amino, cycloalkyl and heterocyclyl;
each R⁶ is identical or different and each is independently selected from the group consisting of hydrogen, halogen, alkyl, haloalkyl, alkoxy, haloalkoxy, cyano, amino, nitro, hydroxy, hydroxyalkyl, cycloalkyl, heterocyclyl, aryl and heteroaryl;
R⁷ is selected from the group consisting of hydrogen, alkyl, haloalkyl, cycloalkyl and heterocyclyl, wherein the alkyl is optionally substituted by one or more substituents selected from the group consisting of halogen, nitro, cycloalkyl and heterocyclyl;
R⁸ and R⁹ are identical or different and are each independently selected from the group consisting of hydrogen, halogen, alkyl, haloalkyl, alkoxy, cyano, amino, nitro, hydroxy and hydroxyalkyl;
R¹⁰ and R¹¹ are identical or different and are each independently selected from the group consisting of hydrogen, alkyl, haloalkyl, amino, hydroxy, hydroxyalkyl, cycloalkyl, heterocyclyl, aryl and heteroaryl;
n is 0, 1, 2, 3 or 4;
s is 0, 1, 2 or 3; and
t is 0, 1, 2 or 3.

2. The compound of formula (I) according to claim 1, being a compound of formula (IA): 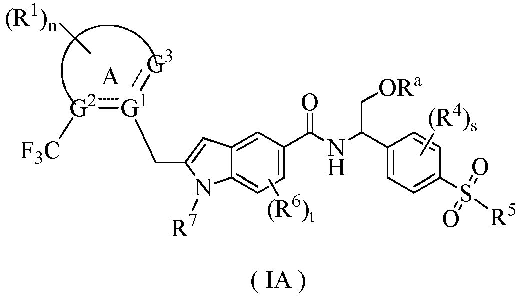 wherein:
R^{a} is hydrogen or alkyl;
-----, ring A, G¹∼G³, R¹, R⁴∼R⁷, n, s and t are as defined in claim 1.

3. The compound of formula (I) according to claim 1 or 2, being a compound of formula (II): 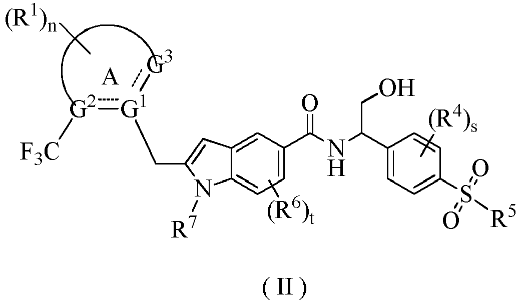 wherein:
-----, ring A, G¹∼G³, R¹, R⁴∼R⁷, n, s and t are as defined in claim 1.

4. The compound of formula (I) according to any one of claims 1 to 3, wherein ring A is selected from the group consisting of phenyl, pyridyl, imidazolyl, pyrazolyl and morpholinyl.

5. The compound of formula (I) according to any one of claims 1 to 4, being a compound of formula (III): 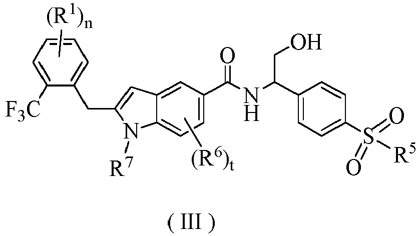 wherein:
R¹, R⁵∼R⁷, n and t are as defined in claim 1.

6. The compound of formula (I) according to any one of claims 1 to 5, being a compound of formula (IV): 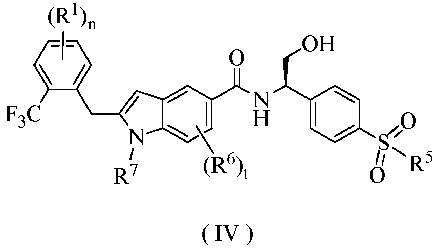 wherein:
R¹, R⁵∼R⁷, n and t are as defined in claim 1.

7. The compound of formula (I) according to any one of claims 1 to 6, wherein R¹ is selected from the group consisting of hydrogen, halogen and alkyl.

8. The compound of formula (I) according to any one of claims 1 to 7, wherein R⁵ is selected from the group consisting of alkyl, NR¹⁰R¹¹ and cycloalkyl, wherein the alkyl and cycloalkyl are each independently optionally substituted by one or more substituents selected from the group consisting of hydroxy, halogen, alkyl, amino, cycloalkyl and heterocyclyl; R¹⁰ and R¹¹ are as defined in claim 1.

9. The compound of formula (I) according to any one of claims 1 to 8, wherein R⁶ is hydrogen or halogen.

10. The compound of formula (I) according to any one of claims 1 to 9, wherein R⁷ is selected from the group consisting of alkyl, cycloalkyl and haloalkyl.

11. The compound of formula (I) according to any one of claims 1 to 10, selected from the group consisting of: 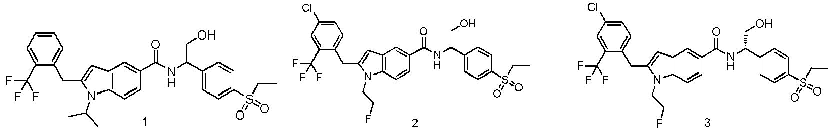 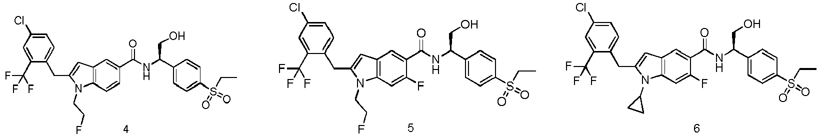 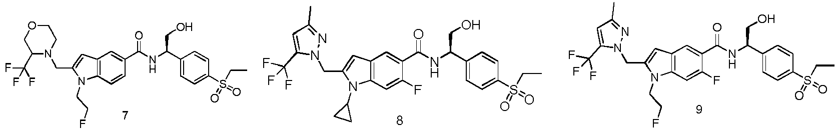 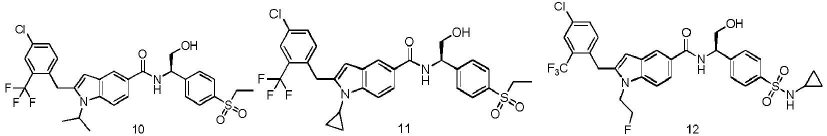 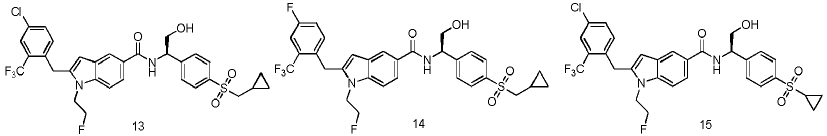 and

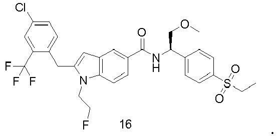

12. A pharmaceutical composition, comprising a therapeutically effective amount of the compound of formula (I) according to any one of claims 1 to 11, and one or more pharmaceutically acceptable carriers, diluents or excipients.

13. The pharmaceutical composition according to claim 12, further comprising an anti-PD-1 antibody.

14. Use of the compound of formula (I) according to any one of claims 1 to 11 or the pharmaceutical composition according to claim 12 in the preparation of a ROR agonist.

15. Use of the compound of formula (I) according to any one of claims 1 to 11 or the pharmaceutical composition according to claim 12 as a ROR agonist in the preparation of a medicament for preventing and/or treating tumor or cancer.

16. Use of the compound of formula (I) according to any one of claims 1 to 11 or the pharmaceutical composition according to claim 12 in combination with an anti-PD-1 antibody in the preparation of a medicament for preventing and/or treating tumor or cancer.
